# EUROPEAN PATENT APPLICATION

(11) **EP 4 737 506 A1**
(43) Date of publication of application: **06.05.2026**
(21) Application number: 24831871.9
(22) Date of filing: 21.06.2024
(51) Int. Cl.: C08G 73/06, G03F 7/023, G09F 9/30, H10K 50/84, H10K 50/86, H10K 59/122, H10K 59/124, H10K 59/173, H10K 59/38, H10K 85/10

(54) **CURED FILM AND ORGANIC EL DISPLAY DEVICE**

(30) Priority: 27.06.2023 JP 2023104748
(71) Applicant: Toray Industries, Inc., Tokyo 103-8666 (JP)
(72) Inventor: KOMORI, Yusuke, Otsu-shi, Shiga 520-8558 (JP); SAEKI, Akinori, Otsu-shi, Shiga 520-8558 (JP); NISHIYAMA, Masahito, Otsu-shi, Shiga 520-8558 (JP)
(74) Representative: Kador & Partner Part mbB
(86) International application number: PCT/JP2024/022588
(87) International publication number: WO 2025/005010

(57) **Abstract**

The present invention relates to a cured film having excellent long-term reliability and suppressing a decrease in luminance during high-temperature continuous operation in particular when used for an organic EL display device. The cured film of the present invention contains a polyimide and/or a polybenzoxazole. The molar ratio F (fluorine atom)/C (carbon atom) of a fluorine atom to a carbon atom obtained when a cross section of the cured film is measured with an electron probe microanalyzer is 0 or more and 0.05 or less.

## Description

### TECHNICAL FIELD

The present invention relates to a cured film and an organic EL display device using the cured film.

### BACKGROUND ART

Many products that use organic electroluminescence (hereinafter, "organic EL") display devices have been developed in display devices including thin displays, such as smartphones, tablet PCs, and televisions.

In general, an organic EL display device has a drive circuit, a planarization layer, a first electrode, a pixel division layer, an organic EL layer, and a second electrode on a substrate. The organic EL display device can emit light when a voltage is applied or an electric current is passed between the first electrode and the second electrode facing each other. As materials for a planarization layer and materials for a pixel division layer among the above-described components, cured films of photosensitive resin compositions are generally used that can be patterned by UV irradiation.

Meanwhile, the demand for high reliability of organic EL display devices has become severer year by year, and materials for a planarization layer and materials for a pixel division layer are also required to have a material that does not undergo a decrease in luminance and pixel shrinkage even after a reliability test under acceleration conditions such as high temperature, high humidity, light irradiation, and a continuous operation. Pixel shrinkage is a phenomenon that the decrease in luminance occurs from edges of each pixel or lighting failure occurs.

The cured films of conventionally proposed positive-type photosensitive resin compositions include a cured film using a composition obtained by mixing a naphthoquinonediazide sulfonic acid ester compound as a photosensitive component with an alkali-soluble resin, the cured film using a polyimide precursor as a resin before curing (see, for example, Patent Document 1), and a polybenzoxazole precursor (see, for example, Patent Document 2).

### PRIOR ART DOCUMENTS

### PATENT DOCUMENTS

Patent Document 1: Japanese Patent Laid-open Publication No. 2002-91343
Patent Document 2: Japanese Patent Laid-open Publication No. 2002-116715
Patent Document 3: International Publication No. 2016-047483

### SUMMARY OF THE INVENTION

### PROBLEMS TO BE SOLVED BY THE INVENTION

However, it is difficult to say that the materials proposed in the above-mentioned Patent Documents have sufficient performance from the viewpoint of long-term reliability. Meanwhile, as materials for a pixel division layer of an organic EL display device, there has also been proposed a positive-type photosensitive resin composition having long-term reliability enhanced by setting a sulfur concentration in a cured film within a certain range (see, for example, Patent Document 3). However, the cured film formed of the resin composition described in Patent Document 3 has improved long-term reliability against light irradiation, but it cannot be said that the cured film sufficiently suppresses a decrease in luminance during high-temperature continuous operation. In view of the above problems, an object of the present invention is to provide a cured film having excellent long-term reliability, and suppressing a decrease in luminance during high-temperature continuous operation in particular when used for an organic EL display device.

### SOLUTIONS TO THE PROBLEMS

In order to solve the above-mentioned problems, a cured film of the present invention has the following configuration.
[1] A cured film containing a polyimide and/or a polybenzoxazole, wherein
   a molar ratio F (fluorine atom)/C (carbon atom) of a fluorine atom to a carbon atom obtained when a cross section of the cured film is measured with an electron probe microanalyzer is 0 or more and 0.05 or less.
[2] The cured film according to [1], further containing one or more compounds selected from the group consisting of a carboxylic acid having an indene structure, a carboxylic acid ester having an indene structure, a sulfonic acid having an indene structure, and a sulfonic acid aryl ester having an indene structure.
[3] The cured film according to [1] or [2], further containing polyhydroxystyrene and/or a polyhydroxystyrene/polystyrene copolymer.
[4] The cured film according to any one of [1] to [3], further containing an aromatic hydrocarbon compound having at least three phenolic hydroxyl groups in one aromatic ring.
[5] The cured film according to any one of [1] to [4], further containing β-alkoxypropionamide.
[6] The cured film according to [1] to [5], further containing one or more sulfonic acids selected from the group consisting of compounds represented by any of the formulas (3) to (5) or sulfonate ions derived from one or more sulfonic acids selected from the group consisting of compounds represented by any of the formulas (3) to (5): 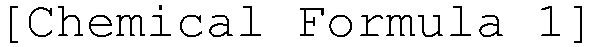 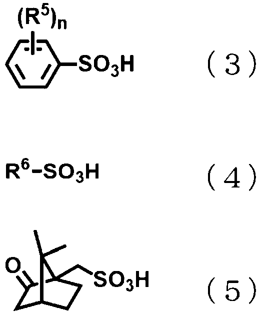 wherein
   in the formula (3), R⁵ each independently represents a hydrogen atom or a monovalent organic group having 1 to 10 carbon atoms, and n represents an integer of 1 to 5; and
   in the formula (4), R⁶ represents a monovalent organic group having 1 to 10 carbon atoms.
[7] The cured film according to [1] to [6], wherein
   the polyimide and/or the polybenzoxazole has repeating units each composed of an acid component and an amine component, and
   when the acid component and the amine component in all the repeating units are each 100 mol%, the total of the acid component having a fluorene structure and the amine component having a fluorene structure is 0 mol% or more and 50 mol% or less with respect to 200 mol% of the total of the acid component and the amine component.
[8] The cured film according to any one of [1] to [7], wherein the polyimide and/or the polybenzoxazole has a residue represented by the formula (6): 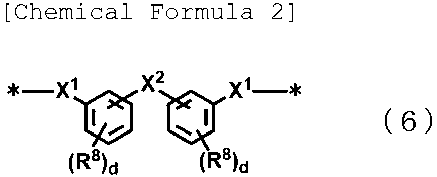 wherein
   in the formula (6), X¹ each independently represents a direct bond, a divalent group represented by the formula (7) or a divalent group represented by the formula (8), X² represents a divalent group represented by the formula (9) or the formula (10), R⁸ each independently represents an alkyl group having 1 to 4 carbon atoms or a hydroxyl group, and d each independently represents an integer of 0 to 4; 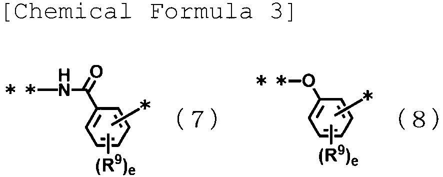
   in the formulas (7) and (8), R⁹ each independently represents an alkyl group having 1 to 4 carbon atoms or a hydroxyl group, e each independently represents an integer of 0 to 4, * represents * in the formula (6), ** represents a bonding point bonded to an aromatic ring, 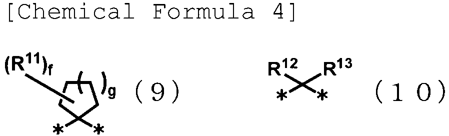
   in the formulas (9) and (10), R¹¹ each independently represents an alkyl group having 1 to 4 carbon atoms, R¹² and R¹³ each independently represent a hydrocarbon group having 1 to 10 carbon atoms or a hydrogen atom, * represents a bonding point bonded to an aromatic ring, f each independently represents an integer of 1 to 4, g represents 1 or 2, provided that R¹² and R¹³ represent different substituents.
[9] The cured film according to any one of [1] to [8], wherein
   the polyimide and/or the polybenzoxazole has repeating units each composed of an acid component and an amine component, and
   when the acid component and the amine component in all the repeating units are each 100 mol%, the total of the acid component having an alicyclic structure and the amine component having an alicyclic structure is 0 mol% or more and 40 mol% or less with respect to 200 mol% of the total of the acid component and the amine component.
[10] The cured film according to any one of [1] to [9], wherein the polyimide and/or the polybenzoxazole contains a repeating unit having a fluorine atom directly bonded to an sp2 carbon.
[11] The cured film according to [10], wherein
   the polyimide and/or the polybenzoxazole has repeating units each composed of an acid component and an amine component, and
   when the acid component and the amine component in all the repeating units are each 100 mol%, the total of the acid component having a fluorine atom directly bonded to the sp2 carbon and the amine component having a fluorine atom directly bonded to the sp2 carbon is 5 mol% or more and 100 mol% or less with respect to 200 mol% of the total of the acid component and the amine component.
[12] The cured film according to any one of [1] to [11], wherein a molar ratio Si (silicon atom)/C (carbon atom) of a silicon atom to a carbon atom obtained when a cross section of the cured film is measured with an electron probe microanalyzer is 0.001 or more and 0.005 or less.
[13] The cured film according to any one of [1] to [12], wherein the cured film has a transmittance of light at a wavelength of 450 nm per film thickness of 2.0 µm of 30% or less.
[14] The cured film according to any one of [1] to [13], wherein the cured film has an OD value of 0.5 to 1.5 in visible light per film thickness of 1 µm.
[15] An organic EL display device including the cured film according to any one of [1] to [14].
[16] The organic EL display device according to [15], further including a color filter having a black matrix.
[17] An electronic component including the cured film according to any one of [1] to [14].

### EFFECTS OF THE INVENTION

The cured film of the present invention has excellent long-term reliability, and suppresses a decrease in luminance during high-temperature continuous operation in particular when used for an organic EL display device.

### BRIEF DESCRIPTION OF THE DRAWINGS

Fig. 1 is a cross-sectional view of an example of an organic EL display device.
Fig. 2 is a cross-sectional view of an example of a display device.
Fig. 3 is a schematic view of the preparation procedure of the organic EL display device.

### EMBODIMENTS OF THE INVENTION

A cured film of the present invention is a cured film containing a polyimide and/or a polybenzoxazole, in which a molar ratio F (fluorine atom)/C (carbon atom) (hereinafter, may be referred to as an F/C ratio) of fluorine to carbon obtained when a cross section of the cured film is measured with an electron probe microanalyzer is 0 or more and 0.05 or less.

The cured film of the present invention is a film of a cured product obtained by curing a composition.

As a result of intensive studies, the present inventors have found that by setting the molar ratio of the fluorine atom to the carbon atom (F/C ratio) in the cured film within the above range, the long-term reliability of the cured film is improved, and a decrease in luminance during high-temperature continuous operation in particular when used for an organic EL display device is suppressed. Although the mechanism is not clear, it is considered that when the F/C ratio in the cured film is within the above range, the gasification of a fluorine component from the cured film and the fluorine contamination of an opening during processing are suppressed, thereby preventing the seepage of the fluorine component into a pixel and the fluorine contamination of an electrode surface particularly in the organic EL display device, and suppressing the decrease in luminance during high-temperature continuous operation.

From the viewpoint of improving the long-term reliability of the cured film and suppressing the decrease in luminance during high-temperature continuous operation in particular when used for the organic EL display device, the F/C ratio obtained when the cross section of the cured film of the present invention is measured with the electron probe microanalyzer is 0 or more and 0.05 or less, preferably 0 or more and 0.03 or less, more preferably 0 or more and 0.02 or less, still more preferably 0 or more and 0.01 or less, and particularly preferably 0. From the viewpoint of improving the reworkability during the preparation of the cured film, the F/C ratio is 0 or more and 0.05 or less, preferably 0.01 or more and 0.05 or less, more preferably 0.02 or more and 0.05 or less, and still more preferably 0.03 or more and 0.05 or less. As a method for measuring the F/C ratio, for example, in the case of an organic EL display device having a cured film, the cured film was exposed by decomposing and polishing the display device, and the peak intensities of fluorine and carbon were measured and determined by a quantitative analysis method using a standard sample using an electron probe microanalyzer.

Furthermore, in the cured film of the present invention, the molar ratio Si (silicon atom)/C (carbon atom) (hereinafter, may be referred to as an Si/C ratio) of a silicon atom to a carbon atom obtained when the cross section of the cured film is measured with the electron probe microanalyzer is preferably 0.001 or more and 0.005 or less. When the Si/C ratio is 0.001 or more, the bending resistance of the cured film can be improved. Although the mechanism is not clear, it is considered that when the Si/C ratio is 0.001 or more, the adhesion of the cured film to a polyimide film substrate is improved, so that the peeling of the cured film at the time of bending is suppressed to improve the bending resistance. Meanwhile, when the Si/C ratio is 0.005 or less, the long-term reliability of the cured film is improved, and the decrease in luminance during high-temperature continuous operation in particular when used for the organic EL display device is suppressed. From the viewpoint of improving the bending resistance of the cured film, the Si/C ratio is preferably 0.001 or more, more preferably 0.002 or more, and still more preferably 0.003 or more. Meanwhile, the Si/C ratio is preferably 0.005 or less, more preferably 0.004 or less, and still more preferably 0.003 or less, from the viewpoint of improving the long-term reliability of the cured film and suppressing the decrease in luminance during high-temperature continuous operation in particular when used for the organic EL display device. The Si/C ratio can be measured in the same manner as in the measurement of the F/C ratio by the electron probe microanalyzer described above. Examples of a method for obtaining a cured film having an Si/C ratio of 0.001 or more and 0.005 or less, the Si/C ratio being obtained when the cross section of the cured film containing the polyimide and/or the polybenzoxazole is measured with the electron probe microanalyzer include, but are not limited to, a method for adjusting the contents of an acid component having a silicon atom and an amine component having a silicon atom in a polyimide and/or a polybenzoxazole described later, and a method for adjusting the content of a silane coupling agent described later.

The cured film of the present invention preferably has a transmittance of light at a wavelength of 450 nm per film thickness of 2.0 µm of 30% or less. When the transmittance of light at a wavelength of 450 nm is 30% or less, in a case where the cured film of the present invention is used for an organic EL display device having an oxide semiconductor thin film transistor, it is possible to prevent the malfunction of the thin film transistor (hereinafter, referred to as a TFT) or the like due to the entry of ultraviolet light into the TFT. In order to prevent the ultraviolet light from entering the TFT, the transmittance of light at a wavelength of 450 nm is preferably 30% or less, more preferably 20% or less, and still more preferably 10% or less. The lower limit of the transmittance of light at a wavelength of 450 nm is not particularly limited, but is 0.01% or more. When the film thickness of the cured film was other than 2.0 µm, the transmittance was converted into absorbance, and the absorbance was corrected to absorbance per film thickness of 2.0 µm. Then, the absorbance was converted into the transmittance again to determine the transmittance of light at a wavelength of 450 nm per film thickness of 2.0 µm.

The cured film of the present invention preferably has an OD value (optical density) of 0.5 to 1.5 in visible light per film thickness of 1 µm. If the OD value is 0.5 or more, the light shielding property can be improved by the cured film, so that when the cured film of the present invention is used for a display device such as an organic EL display device or a liquid crystal display device, external light reflection can be further reduced, allowing to improve contrast in image display. From the viewpoint of reducing reflection, the OD value in visible light per film thickness of 1 µm is preferably 0.5 or more, more preferably 0.6 or more, still more preferably 0.7 or more, and particularly preferably 0.8 or more. If the OD value in visible light per film thickness of 1 µm is 1.5 or less, exposure sensitivity during the preparation of the cured film can be improved. From the viewpoint of high sensitivity, the OD value in visible light per film thickness of 1 µm is preferably 1.5 or less, and more preferably 1.0 or less.

The film thickness of the cured film of the present invention is not particularly limited, but is preferably 1.0 µm or more, more preferably 1.5 µm or more, and still more preferably 2.0 µm or more, from the viewpoint of securing insulation properties. The film thickness of the cured film of the present invention is preferably 5.0 µm or less, more preferably 4.0 µm or less, and still more preferably 3.0 µm or less, from the viewpoint of reducing degassing from the cured film.

The cured film of the present invention is preferably pattern-formed. Since the cured film of the present invention is pattern-formed, it can be suitably used for the application examples of the cured film described later.

Specific examples of a method for obtaining the pattern-formed cured film in which the F/C ratio obtained when the cross section of the cured film containing a polyimide and/or a polybenzoxazole is measured with the electron probe microanalyzer is 0 or more and 0.05 or less include the following.

### [Method 1]

A method for forming a resin film (hereinafter, may be referred to as a resin film 1) having an F/C ratio of 0 or more and 0.05 or less and made of a resin composition containing one or more polymers selected from the group consisting of a polyimide, a polyimide precursor, a polybenzoxazole, a polybenzoxazole precursor, and a copolymer thereof, forming a photosensitive resin film of a photoresist (hereinafter, may be referred to as a photosensitive resin film 1) on the resin film 1, then exposing the photosensitive resin film 1, developing the photosensitive resin film 1, etching the photosensitive resin film 1, removing the photosensitive resin film 1, and heat-treating the resin film 1.

### [Method 2]

A method for forming a photosensitive resin film made of a photosensitive resin composition containing a photosensitive compound and one or more polymers selected from the group consisting of a polyimide, a polyimide precursor, a polybenzoxazole, a polybenzoxazole precursor, and a copolymer thereof, wherein an F/C ratio in a solid excluding a solvent from the photosensitive resin composition is 0 or more and 0.05 or less, and exposing, developing and heat-treating the photosensitive resin film.

Herein, the polyimide precursor refers to a resin which is converted into the polyimide by heat-treatment or chemical-treatment, and examples thereof include a polyamic acid and a polyamic acid ester. The polybenzoxazole precursor refers to a resin which is converted into the polybenzoxazole by heat-treatment or chemical-treatment, and examples thereof include a polyhydroxyamide.

A method for setting the F/C ratio of the cured film to 0 or more and 0.05 or less is not particularly limited, but the method 1 or the method 2 is preferable, and the method 2 is preferable from the viewpoint of simplifying the production step.

### <Polyimide and/or Polybenzoxazole>

The cured film of the present invention contains a polyimide and/or a polybenzoxazole (hereinafter, may be referred to as a component (a)). When the cured film of the present invention contains the component (a), the long-term reliability of the cured film can be improved, and the decrease in luminance during high-temperature continuous operation in particular when used for the organic EL display device can be suppressed.

The component (a) has repeating units each composed of an acid component and an amine component. Here, the acid component and the amine component respectively refer to a repeating unit derived from the acid component and a repeating unit derived from the amine component, which are used as synthetic raw materials of the component (a), among the repeating units constituting the component (a). As the repeating units each composed of an acid component and an amine component, the polyimide has a repeating unit represented by the following formula (1), and the polybenzoxazole has a repeating unit represented by the following formula (2). The cured film of the present invention may contain two or more kinds of each of a resin having the repeating unit represented by the formula (1) and a resin having the repeating unit represented by the formula (2), or may contain a resin obtained by copolymerizing the repeating unit represented by the formula (1) and the repeating unit represented by the formula (2).

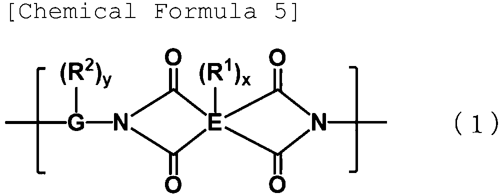

In the formula (1), E represents a tetravalent to decavalent organic group having 4 to 40 carbon atoms, and G represents a divalent to octavalent organic group having 6 to 40 carbon atoms. R¹ and R² each independently represent a carboxy group, a sulfonic acid group, or a hydroxyl group. x and y each independently represent an integer of 0 to 6. Herein, x + y > 0.

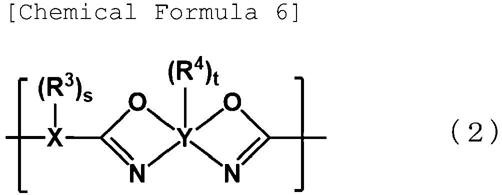

In the formula (2), X represents a divalent to octavalent organic group having 4 to 40 carbon atoms, and Y represents a quadrivalent to decavalent organic group having 6 to 40 carbon atoms. R³ and R⁴ each independently represent a carboxy group, a hydroxyl group, or a sulfonic acid group. s and t each independently represent an integer of 0 to 6.

From the viewpoint of lowering the F/C ratio to 0.05 or less and improving the long-term reliability of the cured film, when the contents of the acid component and the amine component in all the repeating units in the component (a) are each 100 mol%, the total content of the acid component having a fluorine atom and the amine component having a fluorine atom is preferably 0 mol% or more and 50 mol% or less, more preferably 0 mol% or more and 40 mol% or less, still more preferably 0 mol% or more and 20 mol% or less, particularly preferably 0 mol% or more and 10 mol% or less, and most preferably 0 mol%, with respect to 200 mol% of the total content of the acid component and the amine component.

From the viewpoint of setting the Si/C ratio to 0.001 or more and 0.005 or less, and improving the bending resistance of the cured film and the long-term reliability of the cured film, when the contents of the acid component and the amine component in all the repeating units in the component (a) are each 100 mol%, the total content of the acid component having a silicon atom and the amine component having a silicon atom is preferably 2.5 mol% or more and 12.5 mol% or less, more preferably 3 mol % or more and 11 mol% or less, still more preferably 4 mol% or more and 10 mol% or less, and particularly preferably 5 mol% or more and 8 mol% or less, with respect to 200 mol% of the total content of the acid component and the amine component.

From the viewpoint of maintaining the long-term reliability of the cured film while improving the reworkability during the preparation of the cured film, the component (a) preferably contains a repeating unit having a fluorine atom directly bonded to an sp2 carbon. Although the mechanism is not clear, it is considered that the gasification of a fluorine component from the cured film and the fluorine contamination of an opening during processing are suppressed in the case of having the fluorine atom directly bonded to the sp2 carbon as compared with the case of having the fluorine atom directly bonded to an sp3 carbon such as a CF3 group even when the F/C ratio of the cured film is about the same level in addition to the improvement in reworkability involving the improvement in solvent solubility due to the component (a) containing the repeating unit having the fluorine atom.

When the contents of the acid component and the amine component in all the repeating units in the component (a) are each 100 mol%, the total content of the acid component having a fluorine atom directly bonded to the sp2 carbon and the amine component having a fluorine atom directly bonded to the sp2 carbon is preferably 5 mol% or more and 100 mol% or less with respect to 200 mol% of the total content of the acid component and the amine component. From the viewpoint of improving the reworkability during the preparation of the cured film, the total content of the acid component having a fluorine atom directly bonded to the sp2 carbon and the amine component directly bonded to the sp2 carbon is preferably 5 mol% or more, more preferably 10 mol% or more, and still more preferably 20 mol% or more. From the viewpoint of improving the long-term reliability of the cured film, the total content of the acid component having a fluorine atom directly bonded to the sp2 carbon and the amine component directly bonded to the sp2 carbon is preferably 100 mol% or less, more preferably 80 mol% or less, and still more preferably 60 mol% or less.

From the viewpoint of improving the bending resistance of the cured film, when the contents of the acid component and the amine component in all the repeating units in the component (a) are each 100 mol%, the total content of the acid component having a fluorene structure and the amine component having a fluorene structure is preferably 0 mol% or more and 50 mol% or less with respect to 200 mol% of the total content of the acid component and the amine component. From the viewpoint of improving the bending resistance of the cured film, the total content of the acid component having a fluorene structure and the amine component having a fluorene structure is preferably 50 mol% or less, more preferably 30 mol% or less, still more preferably 10 mol% or less, and particularly preferably 0 mol%. Meanwhile, from the viewpoint of improving the reworkability during the preparation of the cured film, the content of the repeating unit having a fluorene structure is preferably 10 mol% or more.

From the viewpoint of improving the solvent solubility of the component (a) and improving the reworkability during the preparation of the cured film, the component (a) preferably has a residue represented by the formula (6).

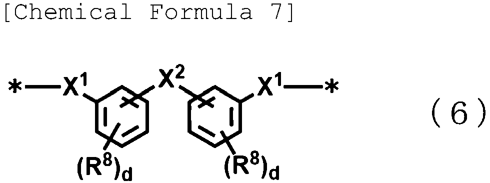

In the formula (6), X¹ each independently represents a direct bond, a divalent group represented by the formula (7) or a divalent group represented by the formula (8), X² represents a divalent group represented by the formula (9) or the formula (10), R⁸ each independently represents an alkyl group having 1 to 4 carbon atoms or a hydroxyl group, and d each independently represents an integer of 0 to 4.

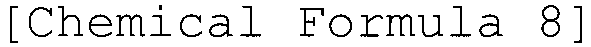

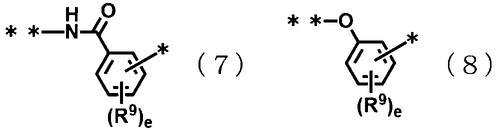

In the formulas (7) and (8), R⁹ each independently represents an alkyl group having 1 to 4 carbon atoms or a hydroxyl group, and e each independently represents an integer of 0 to 4. * represents * in the formula (6), and ** represents a bonding point bonded to an aromatic ring.

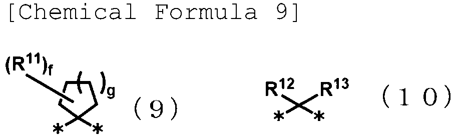

In the formulas (9) and (10), R¹¹ each independently represents an alkyl group having 1 to 4 carbon atoms, R¹² and R¹³ each independently represent a hydrocarbon group having 1 to 10 carbon atoms or a hydrogen atom, and * represents a bonding point bonded to an aromatic ring. f each independently represents an integer of 1 to 4, and g represents 1 or 2. Herein, R¹² and R¹³ represent different substituents.

The residue represented by the formula (6) and contained in the component (a) may be contained in either the acid component or the amine component, but is preferably contained in the amine component since raw materials are easily available.

When the contents of the acid component and the amine component in all the repeating units in the component (a) are each 100 mol%, the content of the residue represented by the formula (6) is preferably 5 mol% or more and 100 mol% or less. From the viewpoint of improving the reworkability during the preparation of the cured film, the content of the residue represented by the formula (6) is preferably 5 mol% or more, more preferably 10 mol% or more, still more preferably 20 mol% or more, and particularly preferably 30 mol% or more. From the viewpoint of improving the bending resistance of the cured film, the content of the residue represented by the formula (6) is preferably 90 mol% or less, more preferably 80 mol% or less, and still more preferably 60 mol% or less.

From the viewpoint of improving the bending resistance of the cured film, when the contents of the acid component and the amine component in all the repeating units in the component (a) are each 100 mol%, the total content of the acid component having an alicyclic structure and the amine component having an alicyclic structure is preferably 0 mol% or more and 40 mol% or less with respect to 200 mol% of the total content of the acid component and the amine component. From the viewpoint of improving the bending resistance of the cured film, the total content of the acid component having an alicyclic structure and the amine component having an alicyclic structure is preferably 40 mol% or less, more preferably 30 mol% or less, still more preferably 20 mol% or less, and particularly preferably 10 mol% or less. Meanwhile, from the viewpoint of improving the reworkability during the preparation of the cured film, the total content of the acid component having an alicyclic structure and the amine component having an alicyclic structure is preferably 10 mol% or more, and more preferably 20 mol% or more.

Examples of a method for measuring whether the cured film contains a polyimide and/or a polybenzoxazole include a method for analyzing the cured film by infrared spectroscopy. In the cured film containing the polyimide and/or the polybenzoxazole, the total content of the acid component having a fluorine atom directly bonded to the sp2 carbon and the amine component having a fluorine atom directly bonded to the sp2 carbon with respect to the total content of the acid component and the amine component in all the repeating units, the total content of the acid component having a fluorene structure and the amine component having a fluorene structure with respect to the total content of the acid component and the amine component in all the repeating units, the content of the residue represented by the formula (6) with respect to the amine component in all the repeating units, and the total content of the acid component having an alicyclic structure and the amine component having an alicyclic structure with respect to the total content of the acid component and the amine component in all the repeating units can be determined, for example, by pyrolysis GC/MS in which the cured film is thermally decomposed at 600°C and then measured by gas chromatography.

The component (a) preferably has 5 to 100,000 repeating units represented by the formula (1) or the formula (2). In addition to the repeating unit represented by the formula (1) or (2), other repeating units may be contained. In this case, the component (a) preferably has 50 mol% or more of the repeating units represented by the formula (1) or the formula (2) with respect to 100 mol% of all the repeating units.

In the above formula (2), X(R³)ₛ represents a residue of an acid. X is a divalent to octavalent organic group having 4 to 40 carbon atoms, and preferably a divalent to octavalent organic group containing an aromatic ring or a cyclic aliphatic group. Examples of the residue of the acid are shown below, but the component (a) may have two or more residues of acids shown below as X(R³)ₛ in the formula (2).

Examples of the residue of the acid containing a fluorine atom include residues of dicarboxylic acids such as 2,2-bis(4-carboxyphenyl)hexafluoropropane, tetrafluoroterephthalic acid, and tetrafluoroisophthalic acid, and residues of 2,2-bis(3,4-dicarboxyphenyl)hexafluoropropane, 2,2-bis(2,3-dicarboxyphenyl)hexafluoropropane, 1,4-difluoro-2,3,4,5-benzenetetracarboxylic acid, 1,4-bis(3.4-dicarboxytrifluorophenoxy)tetrafluorobenzenoic acid, hexafluorooxydiphthalic acid, and an aromatic tetracarboxylic acid having a structure shown below.

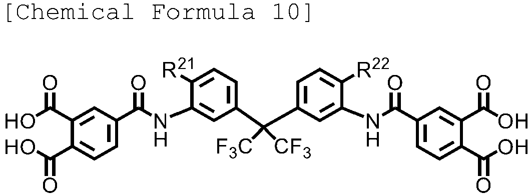

R²¹ and R²² each independently represent a hydrogen atom or a hydroxyl group.

Examples of the residue of the acid containing no fluorine atom include residues of dicarboxylic acids such as terephthalic acid, isophthalic acid, diphenyl ether dicarboxylic acid, biphenylldicarboxylic acid, benzophenonedicarboxylic acid, and triphenyldicarboxylic acid, residues of tricarboxylic acids such as trimellitic acid, trimesic acid, diphenyl ether tricarboxylic acid, and biphenyltricarboxylic acid, and residues of tetracarboxylic acids such as aromatic tetracarboxylic acids (such as pyromellitic acid, 3,3',4,4'-biphenyltetracarboxylic acid, 2,3,3',4'-biphenyltetracarboxylic acid, 2,2',3,3'-biphenyltetracarboxylic acid, 3,3',4,4'-benzophenonetetracarboxylic acid, 2,2',3,3'-benzophenonetetracarboxylic acid, 1,1-bis(3,4-dicarboxyphenyl)ethane, 1,1-bis(2,3-dicarboxyphenyl)ethane, bis(3,4-dicarboxyphenyl)methane, bis(2,3-dicarboxyphenyl)methane, bis(3,4-dicarboxyphenyl)ether, 1,2,5,6-naphthalenetetracarboxylic acid, 2,3,6,7-naphthalenetetracarboxylic acid, 2,3,5,6-pyridinetetracarboxylic acid, 3,4,9,10-perylenetetracarboxylic acid), aromatic tetracarboxylic acids having structures shown below, aliphatic tetracarboxylic acids (such as butanetetracarboxylic acid), and aliphatic tetracarboxylic acids containing a cyclic aliphatic group (such as 1,2,3,4-cyclopentanetetracarboxylic acid).

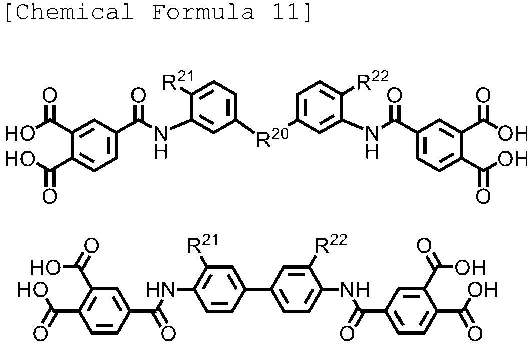

R²⁰ represents an oxygen atom, SO₂, or C(CH₃)₂. R²¹ and R²² each independently represent a hydrogen atom or a hydroxyl group.

In the above formula (1), E(R¹)ₓ represents a residue of an acid dianhydride. E is a tetravalent to decavalent organic group having 4 to 40 carbon atoms, and preferably an organic group containing an aromatic ring or a cyclic aliphatic group. Examples of the residue of the acid dianhydride are shown below, but the component (a) may have two or more residues of acid dianhydrides shown below as E(R¹)ₓ in the formula (1).

Examples of the residue of the acid dianhydride containing a fluorine atom include residues of aromatic tetracarboxylic dianhydrides such as 2,2-bis(3,4-dicarboxyphenyl)hexafluoropropane dianhydride, 1,4-difluoro-2,3,4,5-benzenetetracarboxylic dianhydride, 1,4-bis(3.4-dicarboxytrifluorophenoxy)tetrafluorobenzoic dianhydride, hexafluorooxydiphthalic dianhydride, and an acid dianhydride having a structure shown below.

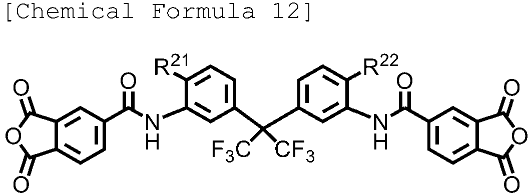

R²¹ and R²² each independently represent a hydrogen atom or a hydroxyl group.

Examples of the residue of the acid dianhydride containing no fluorine atom include residues of aromatic tetracarboxylic dianhydrides such as pyromellitic dianhydride, 3,3',4,4'-biphenyltetracarboxylic dianhydride, 2,3,3',4'-biphenyltetracarboxylic dianhydride, 2,2',3,3'-biphenyltetracarboxylic dianhydride, 3,3',4,4'-benzophenone tetracarboxylic dianhydride, 2,2',3,3'-benzophenone tetracarboxylic dianhydride, 2,2-bis(3,4-dicarboxyphenyl)propane dianhydride, 2,2-bis(2,3-dicarboxyphenyl)propane dianhydride, 1,1-bis(3,4-dicarboxyphenyl)ethane dianhydride, 1,1-bis(2,3-dicarboxyphenyl)ethane dianhydride, bis(3,4-dicarboxyphenyl)methane dianhydride, bis(2,3-dicarboxyphenyl)methane dianhydride, bis(3,4-dicarboxyphenyl)ether dianhydride, 1,2,5,6-naphthalenetetracarboxylic dianhydride, 9,9-bis(3,4-dicarboxyphenyl)fluorenic dianhydride, 9,9-bis{4-(3,4-dicarboxyphenoxy)phenyl}fluorenic dianhydride, 2,3,6,7-naphthalenetetracarboxylic dianhydride, 2,3,5,6-pyridinetetracarboxylic dianhydride, 3,4,9,10-perylenetetracarboxylic dianhydride, and dianhydrides having structures shown below, aliphatic tetracarboxylic dianhydrides such as butanetetracarboxylic dianhydride, and aliphatic tetracarboxylic dianhydrides containing a cyclic aliphatic group, such as 1,2,3,4-cyclopentanetetracarboxylic dianhydride.

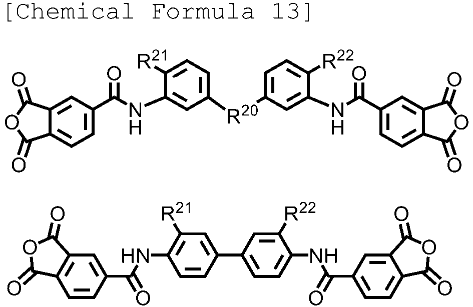

R²⁰ represents an oxygen atom, SO₂, or C(CH₃)₂. R²¹ and R²² each independently represent a hydrogen atom or a hydroxyl group.

Y(R⁴)ₜ in the above formula (2) and G(R²)_{y} in the above formula (1) represent a residue of a diamine or dihydroxydiamine. Y is a tetravalent to decavalent organic group having 6 to 40 carbon atoms, and preferably a tetravalent to decavalent organic group containing an aromatic ring or a cyclic aliphatic group. G is a divalent to octavalent organic group having 6 to 40 carbon atoms, and preferably a divalent to octavalent organic group containing an aromatic ring or a cyclic aliphatic group. Examples of the residues of the diamine and dihydroxydiamine are shown below, but the component (a) may have two or more residues shown below as Y(R⁴)ₜ in the formula (2) and G(R²)_{y} in the formula (1).

Examples of the residues of the diamine and dihydroxydiamine containing a fluorine atom include residues of aromatic diamines such as 2,2'-di(trifluoromethyl)-4,4'-diaminobiphenyl, 2,2'-bis(trifluoromethyl)-5,5'-dihydroxybenzidine, 2,2-bis(3-amino-4-hydroxyphenyl)hexafluoropropane, 2,4,5,6-tetrafluoro-1,3-phenylenediamine, 2,3,5,6-tetrafluoro-1,4-phenylenediamine, 4,4'-diaminooctafluorobiphenyl, 9,9-bis(3-fluoro-4-aminophenyl)fluorene, 2-fluoro-1,4-phenylenediamine, 2,5-difluoro-1,4-phenylenediamine, 4,4'-diamino-2,2'-difluorobiphenyl, 4,4'-diamino-3,3'-difluorobiphenyl, 4,4'-diamino-2,5,2',5'-tetrafluorobiphenyl, 4,4'-diamino-2,6,2',6'-tetrafluorobiphenyl, 4,4'-diaminooctafluorodiphenyl ether, and a compound in which at least some of hydrogen atoms in these aromatic rings are substituted with alkyl groups or halogen atoms, and residues of diamines having structures shown below.

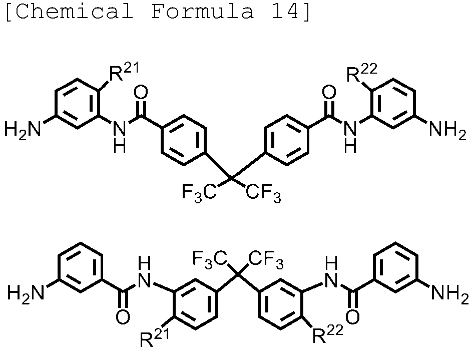

R²¹ to R²² each independently represent a hydrogen atom or a hydroxyl group.

Examples of the residues of the diamine and dihydroxydiamine containing no fluorine atom include residues of aromatic diamine and dihydroxydiamine such as 3,4'-diaminodiphenyl ether, 4,4'-diaminodiphenyl ether, 3,4'-diaminodiphenylmethane, 4,4'-diaminodiphenylmethane, 1,4-bis(4-aminophenoxy)benzene, benzidine, m-phenylenediamine, p-phenylenediamine, 1,5-naphthalenediamine, 2,6-naphthalenediamine, bis(4-aminophenoxy)biphenyl, bis{4-(4-aminophenoxy)phenyl}ether, 1,4-bis(4-aminophenoxy)benzene, 2,2'-dimethyl-4,4'-diaminobiphenyl, 2,2'-diethyl-4,4'-diaminobiphenyl, 3,3'-dimethyl-4,4'-diaminobiphenyl, 3,3'-diethyl-4,4'-diaminobiphenyl, 2,2',3,3'-tetramethyl-4,4'-diaminobiphenyl, 3,3',4,4'-tetramethyl-4,4'-diaminobiphenyl, 9,9-bis(4-aminophenyl)fluorene, 3,5-diaminobenzoic acid, 3,4-diaminobenzoic acid, 2,5-diaminobenzoic acid, bis(3-amino-4-hydroxyphenyl)sulfone, 2,2-bis(3-amino-4-hydroxyphenyl)propane, 4,6-diaminoresorcinol, 9,9-bis(3-amino-4-hydroxyphenyl)fluorene, 3,3'-diamino-[1,1'-biphenyl]-4,4'-diol, and a compound in which at least some of hydrogen atoms in these aromatic rings are substituted with alkyl groups or halogen atoms, residues of aliphatic diamines containing a cycloaliphatic group such as cyclohexyldiamine and methylenebiscyclohexylamine, residues of silicon atom-containing diamines such as 1,3-bis(3- aminopropyl)tetramethyldisiloxane, α,ω-bisaminopolydimethylsiloxane, α,ω-bis(3-aminopropyl)polydimethylsiloxane, 1,3-bis(3-aminopropyl)-1,1,3,3-tetramethyldisiloxane, bis(10-aminodecamethylene)tetramethyldisiloxane, bis(3-aminophenoxymethyl)tetramethyldisiloxane, α,ω-bis(3-aminopropyl)polymethylphenylsiloxane, and α,ω-bis(3-aminopropyl)poly(dimethylsiloxane-diphenylsiloxane)copolymer, residues of diamines having structures shown below, and residues of diamines having the residue represented by the above formula (6).

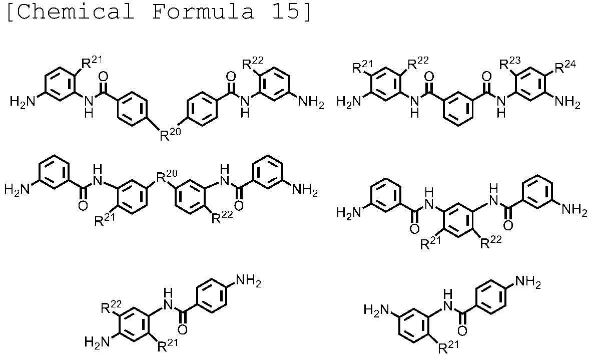

R²⁰ represents an oxygen atom, SO₂, or C(CH₃)₂. R²¹ to R²⁴ each independently represent a hydrogen atom or a hydroxyl group.

From the viewpoint of improving the solvent solubility of the component (a) and improving the reworkability during the preparation of the cured film, X¹ in the formula (6) is preferably a direct bond. Meanwhile, from the viewpoint of improving the sensitivity during the preparation of the cured film, X¹ is preferably a divalent group represented by the formula (7) or a divalent group represented by the formula (8), and more preferably a divalent group represented by the formula (7). X² in the formula (6) represents a divalent group represented by the formula (9) or (10), and preferably has a divalent group represented by the formula (10) from the viewpoint of improving the bending resistance of the cured film. R⁸ each independently represents an alkyl group having 1 to 4 carbon atoms or a hydroxyl group, and from the viewpoint of improving the solvent solubility of the component (a), an alkyl group having 1 to 4 carbon atoms is preferable, and a methyl group is preferable. d each independently represents an integer of 0 to 4, and from the viewpoint of improving the solvent solubility of the component (a), d is preferably an integer of 1 to 4.

In the formulas (7) and (8), R⁹ each independently represents an alkyl group having 1 to 4 carbon atoms or a hydroxyl group, and is preferably a methyl group from the viewpoint that the heat resistance of the component (a) can be enhanced. e each independently represents an integer of 0 to 4, and from the viewpoint of improving the solvent solubility of the component (a), d is preferably an integer of 1 to 4. * represents * in the formula (6), and ** represents a bonding point bonded to an aromatic ring.

In the formula (9), R¹¹ each independently represents an alkyl group having 1 to 4 carbon atoms, and is preferably a methyl group from the viewpoint that the heat resistance of the component (a) can be enhanced. When g represents 1 or 2 and f represents an integer of 1 to 4, both the heat resistance and solvent solubility of the component (a) can be achieved, which is preferable. In the formula (10), R¹² and R¹³ each independently represent a hydrocarbon group having 1 to 10 carbon atoms or a hydrogen atom. From the viewpoint that the heat resistance of the component (a) can be enhanced, R¹² and R¹³ are each independently preferably a saturated hydrocarbon group having 1 to 10 carbon atoms, and more preferably a saturated hydrocarbon group having 1 to 6 carbon atoms. Herein, R¹² and R¹³ represent different substituents. The "different substituents" shown here include those in which the relationship between R¹² and R¹³ is a relationship having a different composition formula, as well as those which are structural isomers having the same composition formula but different bonding states between atoms. When R¹² and R¹³ are different substituents, the solvent solubility of the component (a) can be improved. From the viewpoint of improving the solvent solubility of the component (a), the difference in the number of carbon atoms between R¹² and R¹³ is preferably 1 or more, more preferably 2 or more, and still more preferably 3 or more. Examples of the substituents represented by the formulas (9) and (10) include substituents shown below.

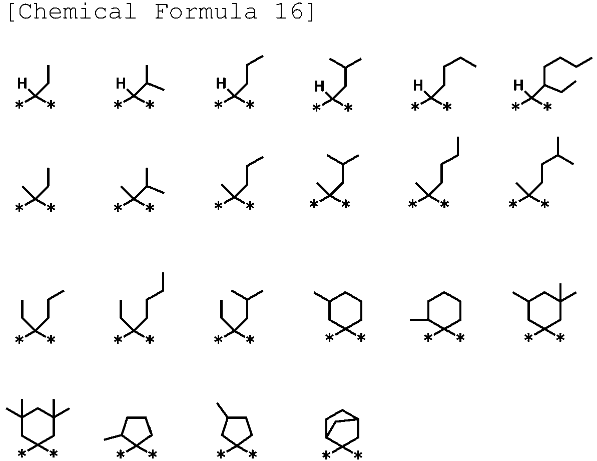

The terminal of these resins may be sealed with a monoamine having a known acidic group, an acid anhydride, an acid chloride, a monocarboxylic acid, and an active ester compound.

The component (a) may be synthesized by a known method, but a solvent containing β-alkoxypropionamide is preferably used as a polymerization solvent. When the polymerization solvent of the component (a) contains β-alkoxypropionamide, the component (a) containing β-alkoxypropionamide is obtained, and a cured film containing β-alkoxypropionamide can be obtained when the component (a) is heat-treated as the resin composition. When the cured film contains β-alkoxypropionamide, the long-term reliability of the cured film can be improved.

The cured film in the present invention may contain a resin other than the component (a). Examples of the resin other than the component (a) include, but are not limited to, a polymer of a radically polymerizable monomer having an acidic group, a cardo resin, a phenol resin, and a siloxane resin.

Examples of the polymer of the radically polymerizable monomer having an acidic group include an acrylic resin, polyhydroxystyrene and/or a polyhydroxystyrene/polystyrene copolymer. As the radically polymerizable monomer having an acidic group, known materials can be used, and examples thereof include o-hydroxystyrene, m-hydroxystyrene, and p-hydroxystyrene, and alkyl, alkoxy-substituted products, methacrylic acid, and acrylic acid thereof, and haloalkyl, alkoxy, halogen, nitro, and cyano substituted products thereof at the α-position.

The cured film of the present invention preferably contains the polyhydroxystyrene and/or the polyhydroxystyrene/polystyrene copolymer from the viewpoint of improving the long-term reliability of the cured film and suppressing the decrease in luminance during high-temperature continuous operation in particular when used for the organic EL display device. In the present invention, the content of the polyhydroxystyrene and/or the polyhydroxystyrene/polystyrene copolymer is preferably 5 parts by mass or more, more preferably 10 parts by mass or more, and still more preferably 15 parts by mass or more, with respect to 100 parts by mass of the component (a). When the content of the polyhydroxystyrene and/or the polyhydroxystyrene/polystyrene copolymer is 5 parts by mass or more with respect to 100 parts by mass of the component (a), the long-term reliability of the cured film can be improved. The content of the polyhydroxystyrene and/or the polyhydroxystyrene/polystyrene copolymer is preferably 60 parts by mass or less, more preferably 50 parts by mass or less, still more preferably 40 parts by mass or less, and particularly preferably 30 parts by mass or less, with respect to 100 parts by mass of the component (a). When the content of the polyhydroxystyrene and/or the polyhydroxystyrene/polystyrene copolymer is 60 parts by mass or less with respect to 100 parts by mass of the component (a), the chemical resistance of the cured film can be maintained.

Examples of a method for confirming whether or not the cured film contains the polyhydroxystyrene and/or the polyhydroxystyrene/polystyrene copolymer include a method in which the cured film is analyzed by infrared spectroscopy, a method in which the cured film is thermally decomposed at 600°C and then analyzed by pyrolysis GC/MS performing measurement by gas chromatography, a method in which components in the cured film are extracted with an organic solvent or the like and the extracted liquid is measured by liquid chromatography, and a method in which components in the cured film are measured by using time-of-flight secondary ion mass spectrometry.

Examples of the cardo resin include a resin having a cardo structure, that is, a skeleton structure in which two cyclic structures are bonded to a quaternary carbon atom constituting a cyclic structure. A common cardo structure is a fluorene ring bonded to a benzene ring.

Examples of the phenol resin include known novolac phenol resins and resol phenol resins obtained by polycondensation of various phenols alone or a mixture of a plurality of phenols thereof with aldehydes such as formalin.

Examples of the siloxane resin include known siloxane resins obtained by hydrolysis and dehydration condensation of one or more types selected from a tetrafunctional organosilane, a trifunctional organosilane, a bifunctional organosilane, and a monofunctional organosilane.

### <Aromatic hydrocarbon compound having at least three phenolic hydroxyl groups in one aromatic ring>

The cured film of the present invention preferably contains an aromatic hydrocarbon compound having at least three phenolic hydroxyl groups in one aromatic ring (hereinafter, may be referred to as a component (b)). When the cured film of the present invention contains the component (b), the long-term reliability of the cured film can be improved, and the decrease in luminance during high-temperature continuous operation in particular when used for the organic EL display device can be suppressed.

Examples of a method for confirming whether or not the cured film contains the component (b) include a method in which the cured film is thermally decomposed at 600°C and then analyzed by pyrolysis GC/MS performing measurement by gas chromatography, a method in which components in the cured film are extracted with an organic solvent or the like and the extracted liquid is measured by liquid chromatography, and a method in which components in the cured film are measured by using time-of-flight secondary ion mass spectrometry.

Examples of the aromatic hydrocarbon structure of the component (b) include known monocyclic and condensed polycyclic structures. The aromatic hydrocarbon has at least three phenolic hydroxyl groups in one aromatic ring. In the present invention, the state having at least three phenolic hydroxyl groups in one aromatic ring represents a state having at least three phenolic hydroxyl groups in a single aromatic ring, and for example, a compound having three aromatic rings having one phenolic hydroxyl group is not included in the component (b) of the present invention. Specific examples of the component (b) include, but are not limited to, a compound having a structure shown below.

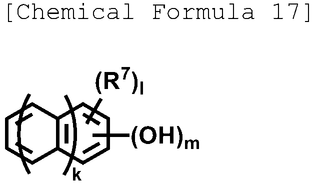

R⁷ independently represents a monovalent organic group having 1 to 20 carbon atoms, k represents an integer of 0 to 2, l represents an integer of 0 to 7, and m represents an integer of 3 to 10. Herein, {(2k + 6) - (1 + m)} ≥ 0.

Examples of the aromatic hydrocarbon having at least three phenolic hydroxyl groups in one aromatic ring include phloroglucinol, pyrogallol, 1,2,4-trihydroxybenzene, 2,4,5-trihydroxybenzaldehyde, 2,3,4-trihydroxybenzaldehyde, 3,4,5-trihydroxybenzaldehyde, gallacetophenone, 2,3,4-trihydroxybenzoic acid, gallic acid, methyl gallate, ethyl gallate, propyl gallate, octyl gallate, 2,3,4-trihydroxybenzophenone, 2,3,4,4'-tetrahydroxybenzophenone, 4,4'-isopropylidenedipyrogallol, 1,2,3,4-tetrahydroxybenzene, 1,2,3,5-tetrahydroxybenzene, 1,2,4,5-tetrahydroxybenzene, and leucoquinizarin.

From the viewpoint of further lowering the transmittance of light of 300 nm to 500 nm of the cured film, that is, from the viewpoint of setting the transmittance of light at a wavelength of 450 nm to 30% or less, in combination with a thermal crosslinking agent described later, as the component (b), with respect to any of the phenolic hydroxyl groups in the component (b), at least one substitution position of other phenolic hydroxyl groups is preferably an ortho position or a para position, and more preferably a para position.

Examples of a compound (b1) in which, with respect to any of the phenolic hydroxyl groups, at least one substitution position of other phenolic hydroxyl groups is the ortho position among the components (b) include pyrogallol, 1,2,4-trihydroxybenzene, 2,4,5-trihydroxybenzaldehyde, 2,3,4-trihydroxybenzaldehyde, 3,4,5-trihydroxybenzaldehyde, gallacetophenone, 2,3,4-trihydroxybenzoic acid, gallic acid, methyl gallate, ethyl gallate, propyl gallate, octyl gallate, 2,3,4-trihydroxybenzophenone, 2,3,4,4'-tetrahydroxybenzophenone, 4,4'-isopropylidenedipyrogallol, 1,2,3,4-tetrahydroxybenzene, 1,2,3,5-tetrahydroxybenzene, and 1,2,4,5-tetrahydroxybenzene. Pyrogallol or 1,2,4-trihydroxybenzene is preferable from the viewpoint of further lowering the transmittance
of light of 300 nm to 500 nm of the cured film in combination with the thermal crosslinking agent described later. Examples of a compound (b2) in which with respect to any of the phenolic hydroxyl groups, at least one substitution position of the other phenolic hydroxyl groups is the para position among the components (b) include 1,2,4-trihydroxybenzene, 2,4,5-trihydroxybenzaldehyde, 1,2,3,4-tetrahydroxybenzene, 1,2,3,5-tetrahydroxybenzene, 1,2,4,5-tetrahydroxybenzene, and leucoquinizarin. From the viewpoint of further lowering the transmittance of light of 300 nm to 500 nm of the cured film in combination with the thermal crosslinking agent described later, 1,2,4-trihydroxybenzene is preferable.

The upper limit of the molecular weight of the component (b) is not particularly limited, but is preferably 1000 or less, more preferably 800 or less, and still more preferably 600 or less. The lower limit of the molecular weight of the component (b) is 126 or more.

In the present invention, the content of the component (b) is preferably 1 part by mass or more, more preferably 5 parts by mass or more, and still more preferably 10 parts by mass or more, with respect to 100 parts by mass of the component (a). By setting the content of the component (b) to 1 part by mass or more with respect to 100 parts by mass of the component (a), the long-term reliability of the cured film of the present invention can be improved. In addition, the content of the component (b) is preferably 50 parts by mass or less, more preferably 40 parts by mass or less, still more preferably 30 parts by mass or less, and particularly preferably 20 parts by mass or less, with respect to 100 parts by mass of the component (a). By setting the content of the component (b) to 50 parts by mass or less with respect to 100 parts by mass of the component (a), the chemical resistance of the cured film can be maintained.

### <Compound derived from photosensitive compound>

The cured film of the present invention preferably contains a compound derived from a photosensitive compound (hereinafter, may be referred to as a component (c)). When the cured film of the present invention contains the compound derived from the photosensitive compound, the step of producing the cured film can be simplified. Examples of the component (c) include a photopolymerization initiator (hereinafter, may be referred to as a component (c1)) and a naphthoquinonediazide compound (hereinafter, may be referred to as a component (c2)).

Examples of the component (c1) include known photopolymerization initiators. Examples thereof include a benzyl ketal compound, an α-hydroxyketone compound, an α-aminoketone compound, an acylphosphine oxide compound, a biimidazole compound, an oxime ester compound, an acridine compound, a titanocene compound, a benzophenone compound, an acetophenone compound, an aromatic ketoester compound, or a benzoic acid ester compound. From the viewpoint of improving the sensitivity during the preparation of the cured film, in the cured film of the present invention, an α-hydroxyketone compound, an α-aminoketone compound, an acylphosphine oxide compound, a biimidazole compound, or an oxime ester compound is preferable, and from the viewpoint of improving the sensitivity at the time of exposure, improving the halftone characteristics, and suppressing the residue after development, an oxime ester compound is more preferable.

Examples of a method for confirming whether or not the cured film contains the compound derived from the component (c) include a method in which the cured film is thermally decomposed at 600°C and then analyzed by pyrolysis GC/MS performing measurement by gas chromatography, a method in which components in the cured film are extracted with an organic solvent or the like and the extracted liquid is measured by liquid chromatography, and a method in which components in the cured film are measured by using time-of-flight secondary ion mass spectrometry.

The content of a compound derived from the component (c1) is preferably 0.1 parts by mass or more, more preferably 1 part by mass or more, and still more preferably 10 parts by mass or more, with respect to 100 parts by mass of the component (a) from the viewpoint of improving the sensitivity during the preparation of the cured film of the present invention. Meanwhile, from the viewpoint of suppressing the residue during the preparation of the cured film of the present invention, the content of the compound derived from the component (c1) is preferably 50 parts by mass or less with respect to 100 parts by mass of the component (a).

Examples of the component (c2) include a known naphthoquinonediazide compound. Examples of the naphthoquinonediazide compound include a 1,2-naphthoquinonediazide-5-sulfonic acid ester compound and/or a 1,2-naphthoquinonediazide-4-sulfonic acid ester compound.

From the viewpoint of improving the long-term reliability of the cured film of the present invention, the compound derived from the photosensitive compound contained in the cured film of the present invention is preferably a compound derived from the component (c2). Specific examples of a compound derived from the naphthoquinonediazide compound include one or more compounds selected from the group consisting of a carboxylic acid having an indene structure, a carboxylic acid ester having an indene structure, a sulfonic acid having an indene structure, and a sulfonic acid aryl ester having an indene structure. One or more compounds selected from the group consisting of a carboxylic acid having an indene structure, a carboxylic acid ester having an indene structure, a sulfonic acid having an indene structure, and a sulfonic acid aryl ester having an indene structure are preferable.

The content of the compound derived from the component (c2) is preferably 0.1 parts by mass or more, more preferably 10 parts by mass or more, and still more preferably 25 parts by mass or more, with respect to 100 parts by mass of the component (a) from the viewpoint of improving the sensitivity during the preparation of the cured film of the present invention. Meanwhile, from the viewpoint of suppressing the residue during the preparation of the cured film of the present invention, the content of the compound derived from the component (c2) is preferably 100 parts by mass or less with respect to 100 parts by mass of the component (a).

### <β-alkoxypropionamide>

The cured film of the present invention preferably contains β-alkoxypropionamide. When the cured film of the present invention contains β-alkoxypropionamide, the long-term reliability of the cured film can be improved, and the decrease in luminance during high-temperature continuous operation in particular when used for the organic EL display device can be suppressed.

Specific examples of a method for obtaining a cured film containing β-alkoxypropionamide include a method in which β-alkoxypropionamide is added to a resin composition for obtaining the cured film of the present invention and the resin composition is heat-treated to obtain the cured film, and a method in which a resin composition containing the component (a) containing β-alkoxypropionamide is heat-treated by using β-alkoxypropionamide as the polymerization solvent of the component (a) to obtain a cured film. From the viewpoint of suppressing the volatilization of β-alkoxypropionamide during heat-treatment and improving the long-term reliability of the cured film, a method in which a resin composition containing the component (a) containing β-alkoxypropionamide is heat-treated by using β-alkoxypropionamide as the polymerization solvent of the component (a) to obtain a cured film is preferred.

Examples of a method for confirming whether or not the cured film contains β-alkoxypropionamide include a method in which components generated from the cured film are thermally decomposed at 600°C by adsorption collection by a purge and trap method, and then analyzed by purge and trap GC/MS performing measurement by gas chromatography.

Examples of the β-alkoxypropionamide include β-methoxypropionamide, β-methoxy-N-methylpropionamide, β-methoxy-N,N-dimethylpropionamide, β-methoxy-N-ethylpropionamide, β-methoxy-N,N-diethylpropionamide, β-methoxy-(N-propyl)propionamide, β-methoxy-(N,N-dipropyl)propionamide, β-methoxy-N-butylpropionamide, β-methoxy-N,N-dibutylpropionamide, β-ethoxy-N,N-dimethylpropionamide, β-propoxy-N,N-dimethylpropionamide, and β-butoxy-N,N-dimethylpropionamide. From the viewpoint of versatility, the β-alkoxypropionamide is preferably β-methoxy-N,N-dimethylpropionamide or β-butoxy-N,N-dimethylpropionamide.

The content of the β-alkoxypropionamide in the cured film is preferably 0.01% by mass or more and 5% by mass or less, more preferably 0.05% by mass or more and 1% by mass or less, and still more preferably 0.1% by mass or more and 0.5% by mass or less, from the viewpoint of improving the long-term reliability of the cured film.

### <Sulfonic acid and/or sulfonate ions>

The cured film of the present invention preferably contains one or more sulfonic acids selected from the group represented by any of the formulas (3) to (5) or sulfonate ions derived from the sulfonic acids (hereinafter, may be referred to as a specific sulfonic acid and/or sulfonate ions).

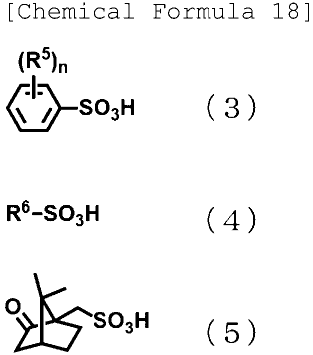

In the formula (3), R⁵ each independently represents a hydrogen atom or a monovalent organic group having 1 to 10 carbon atoms, and n represents an integer of 1 to 5. In the general formula (4), R⁶ represents a monovalent organic group having 1 to 10 carbon atoms.

When the cured film of the present invention contains a specific sulfonic acid and/or sulfonate ions, the long-term reliability of the cured film can be improved, and the decrease in luminance during high-temperature continuous operation in particular when used for the organic EL display device can be suppressed.

Examples of a specific method for obtaining the cured film containing a specific sulfonic acid and/or sulfonate ions include a method in which the cured film is obtained by heat-treating a composition using a compound containing the specific sulfonic acid and/or sulfonate ions, and a method in which the cured film is obtained by heat-treating a composition containing a thermal acid generator that generates a specific sulfonic acid and/or sulfonate ions during heat-treatment, and from the viewpoint of improving the long-term reliability of the cured film, a method in which the cured film is obtained by heat-treating a composition containing a thermal acid generator that generates a specific sulfonic acid and/or sulfonate ions during heat-treatment is preferred.

Examples of a method for confirming whether or not the cured film contains a specific sulfonic acid and/or sulfonate ions include a method in which the cured film is thermally decomposed at 600°C and then analyzed by pyrolysis GC/MS performing measurement by gas chromatography, a method in which components in the cured film are extracted with an organic solvent or the like and the extracted liquid is measured by liquid chromatography, and a method in which components in the cured film are measured by using time-of-flight secondary ion mass spectrometry.

As the thermal acid generator that generates a specific sulfonic acid and/or sulfonate ions during heat-treatment, a thermal acid generator having a sulfonic acid ester structure is preferable, and specific examples thereof include "Irgacure" (registered trademark) PAG103, PAG121 (trade names, manufactured by BASF Japan Ltd.), PA-411, PA-480 (trade names, manufactured by Heraeus K.K.), PAI-01, PAI-101, PAI-106, PAI-1001, PAI-1002, PAI-1003, PAI-1004 (trade names, manufactured by Midori Kagaku Co., Ltd.), SP-082, SP-601, SP-606, SP-607, SP-612 (trade names, manufactured by ADEKA Corporation), NIT, MIN, ILP-110, ILP-110N, ILP-118, ILP-113, PA-223, PA-298 (trade names, manufactured by Heraeus K.K.), NAI-105, NAI-106, NAI-109 (trade names, manufactured by Midori Kagaku Co., Ltd.), and sulfonic acid esters synthesized by a known method using one or more sulfonic acids and alcohols or phenols selected from the group represented by the formula (3) to the formula (5).

In the formula (3), R⁵ each independently represents a hydrogen atom or a monovalent organic group having 1 to 10 carbon atoms, and n represents an integer of 1 to 5. Examples of the monovalent organic group having 1 to 10 carbon atoms include an alkyl group, an alkynyl group, an alkenyl group, an aryl group, and CF₃. From the viewpoint of easy availability of raw materials, R5 is preferably a hydrogen atom or an alkyl group having 1 to 10 carbon atoms, and more preferably a methyl group. From the viewpoint of easy availability of raw materials, n is preferably an integer of 1 to 3, and more preferably 1. Examples of the sulfonic acid represented by the formula (3) include benzenesulfonic acid, toluenesulfonic acid, and styrenesulfonic acid.

In the general formula (4), R⁶ represents a monovalent organic group having 1 to 10 carbon atoms. Examples of the monovalent organic group having 1 to 10 carbon atoms include an alkyl group, an alkynyl group, an alkenyl group, a benzyl group, and CF₃. From the viewpoint of easy availability of raw materials, R⁵ is preferably an alkyl group having 1 to 10 carbon atoms or CF₃, and more preferably an alkyl group having 1 to 10 carbon atoms. Examples of the sulfonic acid represented by the formula (4) include methanesulfonic acid, ethanesulfonic acid, propanesulfonic acid, butanesulfonic acid, octanesulfonic acid, trifluoromethanesulfonic acid, benzylsulfonic acid, and vinylsulfonic acid.

The content of the specific sulfonic acid and/or sulfonate ions is preferably 1 part by mass or more, more preferably 2 parts by mass or more, and still more preferably 3 parts by mass or more, with respect to 100 parts by mass of the component (a) from the viewpoint of improving the long-term reliability of the cured film. Meanwhile, from the viewpoint of improving the heat resistance of the cured film, the content of the specific sulfonic acid and/or sulfonate ions is preferably 15 parts by mass or less, more preferably 10 parts by mass or less, and still more preferably 8 parts by mass or less, with respect to 100 parts by mass of the component (a).

### <Colorant (d)>

The cured film of the present invention preferably further contains a colorant (d) (hereinafter, may be referred to as a component (d)). When the cured film of the present invention contains the component (d), the light shielding property of the cured film can be improved, and external light reflection when used for the display device can be suppressed. Since the cured film of the present invention contains the component (d), the transmittance of light at a wavelength of 450 nm can be reduced, and in a case where the cured film of the present invention is used for an organic EL display device having an oxide semiconductor TFT, the malfunction of the TFT or the like due to the entry of ultraviolet light into the TFT can be prevented. From the viewpoint of improving the visible light shielding property of the cured film and improving the OD value in visible light per film thickness of 1 µm, the component (d) preferably has a maximum absorption wavelength in a wavelength range of 300 nm or more and 850 nm or less. From the viewpoint of setting the transmittance of light at a wavelength of 450 nm to 30% or less, the component (d) preferably has a maximum absorption wavelength in a range of 300 nm or more and 600 nm or less, and more preferably has a maximum absorption wavelength in a range of 400 nm or more and 500 nm or less.

The component (d) preferably contains a dye (d1) and/or a pigment (d2). The component (d) preferably contains at least one or more dyes (d1) or pigments (d2). For example, the component (d) may contain one dye (d1) or pigment (d2), contain two or more dyes (d1) or pigments (d2), or contain one or more dyes (d1) and one or more pigments (d2). The component (d) is preferably a black agent and/or a mixture of two or more colors of colorants.

The component (d) contained in the cured film of the present invention preferably contains the dye (d1) from the viewpoint of solvent solubility. Furthermore, from the viewpoint of improving the sensitivity during the preparation of the cured film of the present invention, the dye (d1) is preferably an ionic dye forming an ion pair of organic ions. In addition, from the viewpoint of increasing the sensitivity and reducing the residue, the component (d) preferably has a sulfonic acid group and/or a sulfonate group.

Examples of the skeleton structure of the dye (d1) include anthraquinone type, azo type, phthalocyanine type, methine type, oxazine type, quinoline type, triarylmethane type, and xanthene type skeleton structures, but are not limited thereto. Among the skeleton structures, an anthraquinone type, azo type, methine type, triarylmethane type, or xanthene type skeleton structure is preferable from the viewpoint of solubility in a solvent and heat resistance. In addition, from the viewpoint of improving the heat resistance, a xanthene type skeleton structure is more preferable. These dyes may be used singly or as a metal-containing complex salt system.

The component (d) contained in the cured film of the present invention preferably contains the pigment (d2) from the viewpoint of improving the heat resistance of the cured film. The pigment (d2) is particularly preferably an organic black pigment, and preferably contains one or more selected from the group consisting of a benzofuranone black pigment, a perylene black pigment, and an azo black pigment.

The content of the component (d) is preferably 0.1 to 300 parts by mass, more preferably 0.2 to 200 parts by mass, and particularly preferably 1 to 200 parts by mass, with respect to 100 parts by mass of the component (a). By setting the content of the component (d) to 0.1 parts by mass or more with respect to 100 parts by mass of the component (a), light having a corresponding wavelength can be absorbed. By setting the content of the component (d) to 300 parts by mass or less, light having a corresponding wavelength can be absorbed while the heat resistance and mechanical characteristic of the cured film are maintained.

### <Compound derived from thermal crosslinking agent>

The cured film of the present invention may contain a compound derived from a thermal crosslinking agent. The term "thermal crosslinking agent" refers to a compound having at least two thermally reactive functional groups such as an alkoxymethyl group, a methylol group, an epoxy group, and an oxetanyl group in the molecule. When the cured film of the present invention contains the compound derived from the thermal crosslinking agent, crosslinking occurs between the thermal crosslinking agent and the component (a) or between the thermal crosslinking agents, so that the heat resistance, chemical resistance, and bending resistance of the cured film can be improved. From the viewpoint of reducing the transmittance of light of 300 nm to 500 nm after curing when used in combination with the component (b) described above, the thermal crosslinking agent preferably has an alkoxymethyl group or a methylol group, and more preferably has an alkoxymethyl group or a methylol group directly substituted with a nitrogen atom. From the viewpoint of reducing the transmittance of the light of 300 nm to 500 nm after curing when used in combination with the component (b) described above, the compound derived from the thermal crosslinking agent preferably has a triazine ring.

The content of the compound derived from the thermal crosslinking agent is preferably 1% by mass or more and 30% by mass or less in 100% by mass of the cured film. When the content of the compound derived from the thermal crosslinking agent is 1% by mass or more, the chemical resistance and bending resistance of the cured film can be further enhanced. When the content of the compound derived from the thermal crosslinking agent is 30% by mass or less, the amount of outgas from the cured film can be further reduced.

### <Compound derived from adhesion improver>

The cured film of the present invention may contain a compound derived from an adhesion improver. As the compound derived from the adhesion improver, known compounds such as a silane coupling agent, a titanium chelate agent, an aluminum chelate agent, and a compound derived from a compound obtained by reacting an aromatic amine compound with an alkoxy group-containing silicon compound can be contained. Two or more of these compounds may be contained. Containing the compound derived from the adhesion improver can enhance the adhesion of the cured film to a base substrate such as a silicon wafer, indium tin oxide (ITO), SiO2, silicon nitride, or a polyimide film substrate.

From the viewpoint of setting the Si/C ratio to 0.001 or more and 0.005 or less, improving the bending resistance of the cured film, and improving the long-term reliability of the cured film, the cured film of the present invention preferably contains a silane coupling agent known as the adhesion improver. Examples of the known silane coupling agent include vinyltrimethoxysilane, vinyltriethoxysilane, 2-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, 3-glycidoxypropyltriethoxysilane, p-styryltrimethoxysilane, 3-methacryloxypropylmethyldimethoxysilane, 3-methacryloxypropyltrimethoxysilane, 3-methacryloxypropyldiethoxysilane, 3-methacryloxypropyltrimethoxysilane, 3-methacryloxypropyldiethoxysilane, 3-methacryloxypropyltrimethoxysilane, 3-acryloxypropyltrimethoxysilane, N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethyl-butylidene)propylamine, N-phenyl-3-aminopropyltrimethoxysilane, 3-ureidopropyltrialkoxysilane, 3-ureidopropyltrimethoxysilane, 3-isocyanatopropyltriethoxysilane, tris-(trimethoxysilylpropyl)isocyanurate, 3-mercaptopropyldimethoxysilane, and 3-mercaptopropyltrimethoxysilane. Among them, from the viewpoint of improving storage stability, one or more silane coupling agents selected from the group consisting of vinyltrimethoxysilane, vinyltriethoxysilane, 2-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, and 3-glycidoxypropyltriethoxysilane are preferable, and 3-glycidoxypropyltrimethoxysilane or 3-glycidoxypropyltriethoxysilane is more preferable.

The content of the adhesion improver is preferably 0.01 to 10% by mass in 100% by mass of the cured film. When the cured film of the present invention contains the known silane coupling agent as the adhesion improver, the content of the known silane coupling agent is preferably 0.6 to 6.0% by mass, more preferably 1.0 to 5.0% by mass, and particularly preferably 2.0 to 4.0% by mass, in 100% by mass of the cured film from the viewpoint of setting the Si/C ratio to 0.001 or more and 0.005 or less, improving the bending resistance of the cured film, and improving the long-term reliability of the cured film.

### <Inorganic particles>

The cured film of the present invention may contain inorganic particles. Preferable specific examples of the inorganic particles include particles made of silicon oxide, titanium oxide, barium titanate, alumina, and talc and the like. The primary particle diameter of the inorganic particles is preferably 100 nm or less, and more preferably 60 nm or less.

The content of the inorganic particles is preferably 5 to 90% by mass in 100% by mass of the cured film.

### <Method for producing cured film>

A method for producing the cured film of the present invention will be described by exemplifying a method in which a photosensitive resin film made of a photosensitive resin composition is formed on a substrate, and the photosensitive resin film is exposed and developed, followed by heat-treating the photosensitive resin film.

First, a step of forming a photosensitive resin film made of a photosensitive resin composition on a substrate will be described.

The photosensitive resin composition to be used for the production of the cured film of the present invention can be obtained by mixing and dissolving, for example, one or more polymers selected from the group consisting of a polyimide, a polyimide precursor, a polybenzoxazole, a polybenzoxazole precursor, and copolymers thereof, a component (c), and if necessary, a component (b), β-alkoxypropionamide, a thermal acid generator that generates a specific sulfonic acid and/or sulfonate ions, a component (d), a thermal crosslinking agent, an adhesion improver, a surfactant, inorganic particles, and a solvent and the like.

Subsequently, the photosensitive resin film can be obtained by applying the photosensitive resin composition onto a base substrate to obtain a coating film of the photosensitive resin composition, and drying the coating film. As the base substrate, a known substrate such as a glass substrate can be used. Examples of the method for applying the photosensitive resin composition of the present invention include a spin coating method, a slit coating method, a dip coating method, a spray coating method, and a printing method.

After the photosensitive resin composition is applied, the photosensitive resin composition is subjected to a vacuum drying treatment if necessary. The vacuum drying rate depends on the volume of a vacuum chamber, the capacity of a vacuum pump, and the diameter of a pipe between the chamber and the pump, and the like, but, for example, is preferably set to a condition that the pressure in the vacuum chamber is reduced to 40 Pa after a lapse of 60 seconds in the absence of the coated substrate.

After the application or vacuum drying, the obtained coating film is generally heated and dried. This step is also referred to as pre-bake. For drying, a hot plate, an oven, or an infrared ray or the like is used. In the case of using the hot plate, the coating film is held and heated directly on the plate, or on a jig such as a proximity pin installed on the plate. The heating time is preferably 1 minute to several hours. The heating temperature depends on the type and the purpose of the coating film, but is preferably 80°C or higher, and more preferably 90°C or higher from the viewpoint of promoting solvent drying at the time of pre-bake. Meanwhile, from the viewpoint of reducing the progress of curing at the time of pre-bake, the heating temperature is preferably 150°C or lower, and more preferably 140°C or lower.

Next, a step of exposing the photosensitive resin film will be described. For example, a desired pattern can be formed by irradiating the photosensitive resin film with actinic rays through a photomask having a desired pattern for exposure and conducting development.

Examples of the actinic rays used for exposure include ultraviolet light, visible light, electron beams, and X-rays. In the present invention, the i-ray (365 nm), the h-ray (405 nm), and the g-ray (436 nm) of a mercury lamp are preferably used. When the photosensitive resin film has positive photosensitivity, the exposed portion is dissolved in the developer. When the photosensitive resin film has negative photosensitivity, the exposed portion is cured and insolubilized in the developer.

Next, a step of developing the exposed photosensitive resin film will be described.

After the exposure, the exposed portion is removed in the case of the photosensitive resin film having positive photosensitivity, and the unexposed portion is removed in the case of the photosensitive resin film having negative photosensitivity with a developer to form a desired pattern. The developer is preferably an aqueous solution of a compound having alkaline properties, such as tetramethylammonium hydroxide, diethanolamine, diethylaminoethanol, sodium hydroxide, potassium hydroxide, sodium carbonate, potassium carbonate, triethylamine, diethylamine, methylamine, dimethylamine, dimethylaminoethyl acetate, dimethylaminoethanol, dimethylaminoethyl methacrylate, cyclohexylamine, ethylenediamine and hexamethylenediamine. To the alkaline aqueous solution, one or more components may be added, and examples of the components include polar solvents such as N-methyl-2-pyrrolidone, N,N-dimethylformamide, N,N-dimethylacetamide, dimethylsulfoxide, γ-butyrolactone, and dimethylacrylamide, alcohols such as methanol, ethanol, and isopropanol, esters such as ethyl lactate and propylene glycol monomethyl ether acetate, and ketones such as cyclopentanone, cyclohexanone, isobutyl ketone, and methyl isobutyl ketone. Examples of the method of development include a spray method, a paddle method, an immersion method, and an ultrasonic method.

Next, the pattern formed by development is preferably rinsed with distilled water. The pattern may be rinsed with distilled water to which a component is added, and examples of the component include alcohols such as ethanol and isopropyl alcohol, and esters such as ethyl lactate and propylene glycol monomethyl ether acetate.

Next, a step of heat-treating the developed photosensitive resin film to obtain a cured film will be described.

The cured film can be obtained by heat-treating the photosensitive resin film developed as described above. When the photosensitive resin composition to be used for producing the cured film of the present invention contains a polyimide precursor or a polybenzoxazole precursor, the cured film containing a polyimide or a polybenzoxazole can be obtained by heat-treatment.

The heat-treatment temperature is preferably 180°C or higher, more preferably 200°C or higher, still more preferably 230°C or higher, and particularly preferably 250°C or higher, from the viewpoint of further reducing the amount of outgas generated from the cured film. Meanwhile, from the viewpoint of improving the film toughness of the cured film, the heat-treatment temperature is preferably 500°C or lower, and more preferably 450°C or lower. In this temperature range, the temperature may be raised stepwise or may be continuously raised. The time for heat-treatment is preferably 30 minutes or more from the viewpoint of further decreasing the amount of outgas. From the viewpoint of improving the film toughness of the cured film, the time for heat-treatment is preferably 3 hours or less. Examples thereof include a method for heat-treating at 150°C and 250°C for 30 minutes each, and a method for heat-treatment while linearly increasing the temperature from room temperature to 300°C over a period of 2 hours.

An atmosphere during heat-treatment is preferably under a low oxygen concentration of less than 5% from the viewpoint of improving the long-term reliability of the cured film and suppressing the decrease in luminance during high-temperature continuous operation in particular when used for the organic EL display device. Examples of an inert gas for setting the oxygen concentration to less than 5% include nitrogen and argon. The oxygen concentration in the inert gas atmosphere is preferably less than 5%, more preferably less than 1%, still more preferably less than 0.5%, and particularly preferably less than 0.01%.

### <Application example of cured film>

The cured film of the present invention is suitably used for a surface protective layer or an interlayer insulation layer of a semiconductor element, a pixel division layer of an organic EL element, a planarization layer of a TFT substrate of a display device using the organic EL element, a wiring protection insulation layer of a circuit board, an on-chip microlens of a solid-state imaging device, and a planarization layer for various display devices/solid-state imaging devices, and the like. For example, the cured film is suitable as a surface protective layer or an interlayer insulation layer in an MRAM having low heat resistance or in a promising next-generation memory such as a polymer memory (polymer ferroelectric RAM: PFRAM) or a phase change memory (phase change RAM: PCRAM or ovonics unified memory: OUM).

### <Organic EL display device>

The organic EL display device of the present invention includes the cured film of the present invention. The organic EL display device using the cured film of the present invention includes at least a substrate, a first electrode, a second electrode, an organic EL layer, a planarization layer, and a pixel division layer, and the cured film is preferably included in the planarization layer and/or the pixel division layer. When the planarization layer and/or the pixel division layer includes the cured film of the present invention, it is possible to provide the organic EL display device having excellent long-term reliability.

The organic EL display device using the cured film of the present invention is an organic EL display device having multiple pixels formed on a matrix. The driving system of the organic EL display device is roughly divided into a passive matrix type in which electrodes are divided into columns and rows and only pixels sandwiched between the electrodes emit light and an active matrix type in which several TFTs are provided in each pixel and switching is performed, but is not particularly limited. In the organic EL display device, a planarization layer, a first electrode, a pixel division layer, an organic EL layer, and a second electrode are formed in this order on a substrate. The active matrix type organic EL display device has, on a substrate such as glass, a TFT and a drive circuit that includes a wiring located at the lateral side of the TFT and connected to the TFT, and has a planarization layer on the drive circuit to cover recesses and protrusions, furthermore, with an organic EL layer provided on the planarization layer. The organic EL layer and the wiring are connected to each other through a contact hole formed in the planarization layer. In the organic EL display device according to the embodiment of the present invention, the pixel division layer is formed on the first electrode.

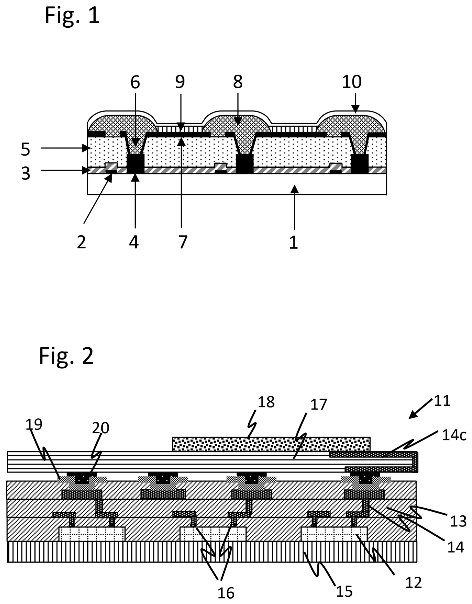

Fig. 1 shows a cross-sectional view of an organic EL display device provided on a substrate 1. Bottom gate type or top gate type TFTs 2 are provided in lines on the substrate 1, and a TFT insulation layer 3 is formed so as to cover the TFTs 2. Wirings 4 respectively connected to the TFTs 2 are provided beneath the TFT insulation layer 3. On the TFT insulation layer 3, a planarization layer 5 is further provided. Contact holes 6 are provided in the planarization layer 5 so that the wirings 4 are exposed. Further, a first electrode 7 is formed on the planarization layer 5 to be connected to the wirings 4 through the contact holes 6. Further, a pixel division layer 8 is formed so as to cover the peripheral edge of the first electrode 7. Furthermore, an organic EL layer 9 and a second electrode 10 are formed thereon. This organic EL display device may be a top emission type device where emitted light is emitted from the side opposite to the substrate 1, or may be a bottom emission type device where light is extracted from the substrate 1 side.

In the organic EL display device, a color display can be obtained by arranging organic EL elements having luminescence peak wavelengths in red, green, and blue regions, respectively, as the organic EL layer, or by providing a white organic EL layer on the entire surface and using it in combination with a color filter. In the color display, normally, the peak wavelength of light in the red region to be displayed is in a range of 560 to 700 nm, the peak wavelength of light in the green region is in a range of 500 to 560 nm, and the peak wavelength of light in the blue region is in a range of 420 to 500 nm.

In the organic EL element, each of the ranges in which the first electrode and the second electrode, which are placed opposite to each other, intersect with each other and overlap with each other and which are regulated by the pixel division layer on the first electrode is referred to as a light-emitting pixel. The shape of the light-emitting pixel is not particularly limited, and may be, for example, rectangular or circular, and can be easily changed depending on the shape of the pixel division layer. In an active matrix type display, an area in which a switching means is formed may be arranged so as to occupy a part of the light-emitting pixel, and thus the light-emitting pixel may have a partially missing shape rather than a rectangular shape.

In the preparation of the organic EL element of the present invention, an organic EL layer is formed by a mask evaporation method. The mask evaporation method is a method in which an organic compound is evaporated using an evaporation mask to perform patterning, wherein the evaporation is carried out while placing an evaporation mask having a desired pattern as an opening section on the evaporation source side of a substrate. In order to produce a high-precision evaporation pattern, it is important to closely adhere an evaporation mask having high flatness onto a substrate. In this case, a technique in which a tension is applied to the evaporation mask, or a technique in which an evaporation mask is closely adhered onto a substrate with a magnet that is placed on the back surface of the substrate or the like is generally employed.

Examples of the method for producing the evaporation mask having a desired pattern as an opening section include an etching method, mechanical polishing, a sandblasting method, a sintering method, a laser processing method, and the utilization of a photosensitive resin and the like. In the case where a fine pattern is required, an etching method and an electroforming method each capable of achieving excellent processing precision are commonly employed.

The configuration of the organic EL layer included in the organic EL element of the present invention is not particularly limited, and may be any one of the following configurations: (1) a hole transport layer/a light-emitting layer; (2) a hole transport layer/a light-emitting layer/an electron transport layer; and (3) a light-emitting layer/an electron transport layer.

Subsequently, a second electrode is formed. In an active matrix type, the second electrode is often formed solidly over the whole area of the light-emitting area. The second electrode is required to have a function of a cathode, i.e., a function of injecting electrons efficiently. Therefore, a metal material is usually used for the second electrode, from the viewpoint of the stability of the electrode. Alternatively, it is also possible to utilize the first electrode as a cathode and utilize the second electrode as an anode.

After the second electrode is formed, sealing is performed to obtain an organic EL display device. In general, an organic EL element is believed to be sensitive to oxygen and moisture. Therefore, in order to produce a display device having high reliability, it is preferred to carry out the sealing under an atmosphere having smaller oxygen and moisture concentrations as possible. With respect to the member to be used for the sealing, it is preferred to select a member having high gas barrier property.

The organic EL display device of the present invention preferably further includes a color filter having a black matrix in order to enhance the effect of reducing external light reflection. The black matrix preferably contains, for example, a resin such as an epoxy resin, an acrylic resin, a urethane resin, a polyester resin, a polyimide resin, a polyolefin resin, or a siloxane resin.

The black matrix contains a colorant. Examples of the colorant that can be contained include black organic pigments, mixed organic pigments, and inorganic pigments. Examples of the black organic pigments that can be contained include carbon black, perylene black aniline black, and benzofuranone pigments. Examples of the mixed organic pigments that can be contained include pigments produced by mixing two or more pigments of a color of red, blue, green, purple, yellow, magenta, and/or cyan to make a pseudo black color. Examples of the black inorganic pigments that can be contained include graphite; fine particles of metals such as titanium, copper, iron, manganese, cobalt, chromium, nickel, zinc, calcium, and silver; metal oxides; metal composite oxides, metal sulfides, metal nitrides; metal oxynitrides; and metal carbides. Among them, carbon black, titanium nitride, and titanium carbide having high light shielding property, and composite particles of these and a metal such as silver are preferable.

The OD value of the black matrix is preferably 1.5 or more, more preferably 2.5 or more, and still more preferably 4.5 or more.

### <Display device other than organic EL display device>

A display device of another aspect having the cured film of the present invention will be described with reference to Fig. 2 as an example. This display device includes at least a metal wiring, the cured film of the present invention, and a plurality of light-emitting elements, in which each of the light-emitting elements includes a pair of electrode terminals on either one surface, the pair of electrode terminals is connected to a plurality of the metal wirings extending in the cured film, and the plurality of the metal wirings are configured to retain electrical insulation properties by the cured film.

In Fig. 2, a display device 11 includes a plurality of light-emitting elements 12 disposed on a counter substrate 15 and a cured film 13 disposed on the light-emitting elements 12. The phrase "on the light-emitting element" may be not only "on the surface of the light-emitting element" but also "on the upper side of the support substrate or the light-emitting element". In the aspect shown in Fig. 2, a configuration is exemplified in which a plurality of cured films 13 are further laminated on the cured film 13 placed so as to be in contact with at least a part of the light-emitting element 12 and a total of three cured films are laminated, but the cured film 13 may be a single layer. The light-emitting element 12 includes a pair of electrode terminals 16 on a surface opposite to a surface in contact with the counter substrate 15, and each electrode terminal 16 is connected to the metal wiring 14 extending in the cured film 13. When a plurality of metal wirings 14 extending in the cured film 13 are covered with the cured film 13, the cured film 13 also functions as an insulation layer, so that the metal wirings are configured to retain electrical insulation properties. The fact that the metal wiring is configured to retain electrical insulation properties means that a portion requiring electrical insulation properties of the metal wiring is covered with the cured film containing the component (a). In the present invention, the state that the insulation layer has electrical insulation properties means a state that the volume resistivity of the insulation layer is 10¹² Ω·cm or more. The light-emitting element 12 is electrically connected to a drive element 18 added to a light-emitting element drive substrate 17 provided at a position facing the counter substrate 15 through the metal wirings 14 and 14c, and the light emission of the light-emitting element 12 can be controlled. The light-emitting element drive substrate 17 is electrically connected to the metal wiring 14 via, for example, a solder bump 20. In order to prevent diffusion of metal such as the metal wiring 14, a barrier metal 19 may be disposed.

### <Electronic component>

An electronic component of the present invention is an electronic component having the cured film of the present invention. The cured film of the present invention can be used for an electronic component such as a semiconductor device. Specific examples of the electronic component include active components having a semiconductor such as transistors, diodes, integrated circuits (ICs), and memories, as well as passive components such as resistors, capacitors, and inductors. Further, an electronic component using a semiconductor is also referred to as a semiconductor device or a semiconductor package. The semiconductor device mentioned in the present invention refers to a general device that can function by utilizing characteristics of a semiconductor element. An electro-optical device or a semiconductor circuit board in which a semiconductor element is connected to a substrate, a device in which a plurality of semiconductor elements are laminated, and an electronic device including these are all included in the semiconductor device. In addition, electronic components such as an interposer for connecting a semiconductor element to a substrate are also included in the semiconductor device. Specifically, the cured film of the present invention has excellent electrical insulation properties, mechanical strength, adhesiveness, and heat resistance, and thus can be used as a surface protective film such as a passivation film or a buffer coat film of the semiconductor element, an interlayer insulation film between redistribution layers formed on the surface of the semiconductor element, an insulation film between elements when a plurality of semiconductor elements are bonded, a multilayer wiring board for high-density packaging, or an insulation film between wiring layers of an interposer.

More preferably, the semiconductor device is a semiconductor device in which the cured film of the present invention is disposed as a surface protective film of a semiconductor or an interlayer insulation film between redistribution layers. By disposing the cured film of the present invention as the surface protective film of the semiconductor or the interlayer insulation film between the redistribution layers, the semiconductor device having high reliability can be obtained.

More preferably, the semiconductor device is a semiconductor device in which the two to ten redistribution layers and interlayer insulation films are repeatedly disposed. The two to ten redistribution layers and interlayer insulation films are repeatedly disposed, whereby the semiconductor device can be downsized.

The electronic component of the present invention preferably includes the cured film of the present invention on the substrate. The substrate on which the cured film is formed can be appropriately selected depending on applications and steps, and examples thereof include a silicon substrate, a silicon carbide substrate, a gallium nitride substrate, ceramics, gallium arsenide, metal, and an epoxy resin. The substrate is preferably a silicon substrate, a silicon carbide substrate, or a gallium nitride substrate, and more preferably a silicon carbide substrate or a gallium nitride substrate.

### EXAMPLES

Hereinafter, the present invention will be described with reference to Examples and the like, but the present invention is not limited to these examples. Each evaluation in Examples was performed with the following method.

### (1) Electron probe microanalyzer measurement of cured film

A varnish obtained in each of Examples and Comparative Examples was applied onto a glass substrate of 5 centimeter square by spin coating so that the film thickness after heat-treatment (curing) was 2.0 µm, and pre-baked at 120°C for 120 seconds to prepare a pre-baked film. Thereafter, without performing UV exposure, development was performed with a 2.38% aqueous tetramethylammonium hydroxide (hereinafter, TMAH) solution so that the film loss of an unexposed portion was about 0.5 µm. Then, the pre-baked film was cured at 250°C for 60 minutes under a nitrogen atmosphere using a high-temperature clean oven INH-9CD-S manufactured by Koyo Thermo Systems Co., Ltd. to prepare a cured film.

The film thickness of the cured film was measured using a stylus profiler (P-15, manufactured by KLA Corporation).

The cross section of the cured film thus obtained was exposed by oblique polishing and ion milling. The exposed cross section of the cured film was subjected to carbon evaporation, and then subjected to elemental analysis with an electron probe microanalyzer JXA-8530F (manufactured by JEOL Ltd.). The measurement conditions were as follows: accelerating voltage: 6 kV, irradiation current: 25 nA, and measurement time: 10 seconds. A Kα peak intensity of 44.00 angstroms was measured for C (carbon atom) using an LDE2H analyzing crystal, a Kα peak intensity of 7.12 angstroms was measured for Si (silicon atom) using a PETH analyzing crystal, a Kα peak intensity of 31.60 angstroms was measured for N (nitrogen atom) using an LDE1 analyzing crystal, a Kα peak intensity of 23.71 angstroms was measured for O (oxygen atom) using an LDE1 analyzing crystal, and a Kα peak intensity of 18.31 angstroms was measured for F (fluorine atom) using an LDE1 analyzing crystal. As the standard samples of the respective elements, BaSO₄, SiO₂, AlN, CaF₂, and SiC were used, and the samples were subjected to ZAF correction (Z: atomic number correction, A: absorption correction, F: fluorescence excitation correction). Each sample was measured 3 times, the mol% of each element in the cured film was calculated from the average value to calculate an F/C ratio, which is the molar ratio of a fluorine atom to a carbon atom, and an Si/C ratio, which is the molar ratio of a silicon atom to a carbon atom.

### (2) Evaluation of reworkability during preparation of cured film

The developed resin film-equipped glass substrate, which was prepared in the same manner as in (1), was immersed in propylene glycol monomethyl ether, and allowed to stand at room temperature for 30 minutes. Thereafter, the glass substrate was taken out from the propylene glycol monomethyl ether, and the presence or absence of a residue on the glass substrate was visually observed. A case where no residue was observed on the glass substrate was determined as "A", a case where a residue was slightly observed on the glass substrate was determined as "B", and a case where a large amount of residue was observed on the glass substrate was determined as "C".

(3) Evaluation of bending resistance of cured film The varnish obtained in each of Examples and Comparative Examples was applied onto a polyimide film substrate by spin coating so that the film thickness after heat-treatment (curing) was 2.0 µm, and pre-baked at 120°C for 120 seconds to prepare a pre-baked film. Thereafter, without performing UV exposure, development was performed with a 2.38% aqueous TMAH solution so that the film loss of an unexposed portion was about 0.5 µm. Then, the pre-baked film was cured at 250°C for 60 minutes under a nitrogen atmosphere using a high-temperature clean oven INH-9CD-S manufactured by Koyo Thermo Systems Co., Ltd. to prepare a cured film.

Subsequently, 10 polyimide film substrates each provided with the cured film were cut into a size of 50 mm in length × 10 mm in width. Next, with the surface of the cured film facing outward, the polyimide film substrate was held for 30 seconds in a state where the substrate was bent in a range of a radius of curvature of 0.1 to 1.0 mm on a 25 mm vertical line. After 30 seconds, the bent polyimide film substrate was restored to the original state, the bent part on the 25 mm vertical line of the cured film surface was observed with the use of an FPD inspection microscope (MX-61L; manufactured by Olympus Corporation), and the appearance change of the cured film surface was evaluated. The bending test was performed while the radius of curvature was changed within a range of a radius of curvature of 0.1 to 1.0 mm, and the minimum radius of curvature at which peeling of the cured film from the polyimide film substrate and a change in appearance such as cracks in the surface of the cured film did not occur was recorded. A case where the minimum radius of curvature was less than 0.2 mm was determined as "S", a case where the minimum radius of curvature was 0.2 mm or more and less than 0.4 mm was determined as "A", a case where the minimum radius of curvature was 0.4 mm or more and less than 0.6 mm was determined as "B", and a case where the minimum radius of curvature was 0.6 mm or more was determined as "C".

### (4) Calculation of content of β-alkoxypropionamide in cured film

10 mg of the cured film prepared by the same method as that in (1) was collected, and a component containing β-alkoxypropionamide was adsorbed and collected by a purge and trap method. Specifically, the collected cured film was heated at 400°C for 60 minutes using helium as a purge gas, and the component desorbed from the cured film was collected in an adsorption tube. The collected component was desorbed from the adsorption tube by heating at 320°C for 5 minutes, and subjected to GC-MS analysis under the conditions of a column temperature: 40°C to 300°C, a carrier gas: helium (1.5 mL/min), and a scanning range: m/Z = 29 to 600 using a GC-MS apparatus 7890/5975C (manufactured by Agilent). The same β-alkoxypropionamide as the detected β-alkoxypropionamide was used as a standard substance, and GC-MS analysis was performed under the same conditions as described above to create a calibration curve, thereby calculating the gas generation amount of β-alkoxypropionamide. The content (% by mass) of β-alkoxypropionamide in the cured film was calculated from the gas generation amount of β-alkoxypropionamide thus obtained and the mass of the sample used for measurement.

### (5) Evaluation of long-term reliability of organic EL display device (evaluation of luminance during high-temperature continuous operation)

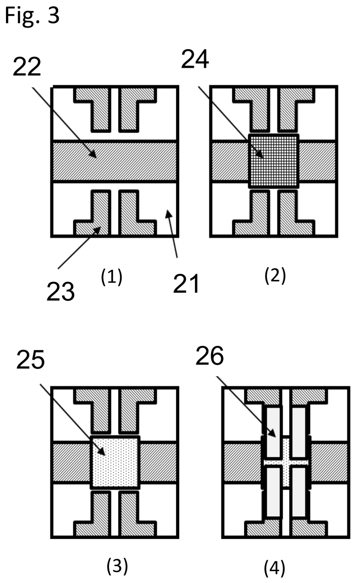

Fig. 3 shows a schematic view of the preparation procedure of the organic EL display device. First, an ITO transparent conductive film having a thickness of 10 nm was formed on the entire surface of an alkali-free glass substrate 21 having a size of 38 mm × 46 mm by a sputtering method, and then etched into a desired shape to form a first electrode (transparent electrode) 22. At the same time, an auxiliary electrode 23 configured to take out a second electrode was also formed (Fig. 3 (1)). The obtained electrode-equipped substrate was ultrasonically cleaned with Semico Clean 56 (trade name, manufactured by Furuuchi Chemical Corporation) for 10 minutes, and then cleaned with ultrapure water. Next, the photosensitive resin composition obtained in each of Examples and Comparative Examples was applied onto the entire surface of the substrate by a spin coating method, and then pre-baked on a hot plate at 120°C for 2 minutes. The obtained pre-baked film was exposed from a high-pressure mercury lamp as a light source with the minimum exposure dose of each photosensitive resin composition through a photomask having a desired pattern, then developed with a 2.38% by mass aqueous TMAH solution to dissolve an unnecessary portion, and rinsed with pure water to obtain a resin pattern. The obtained resin pattern was heat-treated and cured at 230°C for 60 minutes under an air atmosphere or a nitrogen atmosphere using a high-temperature clean oven INH-9CD-S manufactured by Koyo Thermo Systems Co., Ltd. In this way, opening sections of 70 µm in width and 260 µm in length were arranged at a pitch of 155 µm in the width direction and a pitch of 465 µm in the length direction, and a pixel division layer 24 in a shape for exposing the first electrode through the respective opening sections was formed only on a substrate effective area in a limited fashion. In this way, a pixel division layer having an aperture ratio of 25% was formed in the substrate effective area having a quadrangular shape having one side length of 16 mm. The pixel division layer had a thickness of about 1.5 µm (Fig. 3 (2)).

Next, an organic EL layer 25 including a light-emitting layer was formed on the substrate on which the pixel division layer was formed by a vacuum evaporation method. The degree of vacuum during the evaporation process was 1 × 10⁻³ Pa or less, and the substrate was rotated relative to an evaporation source during the evaporation process. First, a compound (HT-1) was evaporated at a thickness of 10 nm as a hole injection layer, and a compound (HT-2) was evaporated at a thickness of 50 nm as a hole transport layer. Next, a compound (GH-1) that served as a host material and a compound (GD-1) that served as a dopant material were evaporated as a light-emitting layer at a thickness of 40 nm in such a manner that the doping concentration became 10%. Next, a compound (ET-1) and a compound (LiQ) that served as electron transport materials were laminated at a thickness of 40 nm and at a volume ratio of 1 : 1. In this way, the organic EL layer 25 including the hole injection layer/the hole transport layer/the light-emitting layer/the electron transport was formed (Fig. 3(3)). The structures of the compounds used for the organic EL layer are as follows.

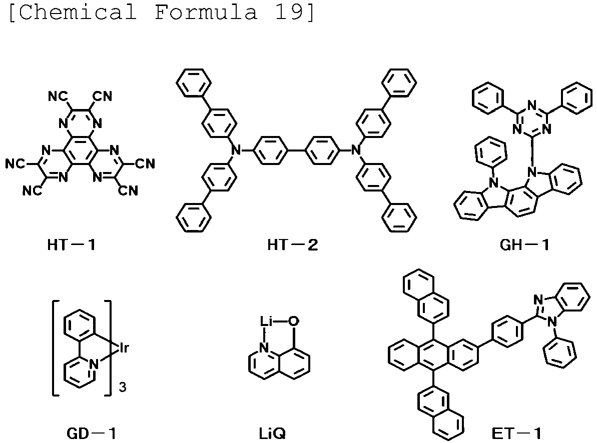

Next, a compound (LiQ) was evaporated at a thickness of 2 nm, and then Mg and Ag were evaporated at a thickness of 10 nm and at a volume ratio of 10 : 1 to form a second electrode (non-transparent electrode) 26 (Fig. 3(4)). Finally, a cap-shaped glass plate was bonded using an epoxy resin adhesive under a low-humidity nitrogen atmosphere for sealing, and thus four top-emission organic EL display devices each having a quadrangular shape having one side length of 5 mm were prepared on one substrate. The term "film thickness" as used herein refers to a displayed value on a quartz oscillation-type film thickness monitor.

By the above method, the prepared organic EL display device was DC driven at 10 mA/cm², and the initial luminance was measured. Next, the same organic EL display device was continuously DC driven at 10 mA/cm² at 85°C, and a time until the luminance decreased to half from the initial luminance was measured as a half-life of luminance. A case where the half-life of luminance was 400 hours or more was determined as "S", a case where the half-life of luminance was 380 hours or more and less than 400 hours was determined as "A", a case where the half-life of luminance was 360 hours or more and less than 380 hours was determined as "B", and a case where the half-life of luminance was less than 360 hours was determined as "C".

### (6) Evaluation of light shielding property at 450 nm (transmittance at 450 nm)

For the cured film prepared by the same method as that in (1), the transmission spectrum of light having a wavelength of 300 nm to 800 nm was measured using a UV-visible spectrophotometer MultiSpec-1500 (manufactured by Shimadzu Corporation), and the transmittance of light at a wavelength of 450 nm per film thickness of 2.0 µm after curing was determined.

When the film thickness of the cured film was other than 2.0 µm, the transmittance was converted into absorbance, and the absorbance was corrected to absorbance per film thickness of 2.0 µm. Then, the absorbance was converted into the transmittance again to determine the transmittance at a wavelength of 450 nm per film thickness of 2.0 µm.

### (7) Evaluation of visible light shielding property (OD value per 1 µm)

The OD value of the cured film prepared in the same manner as in (1) was measured using an optical densitometer (361T; manufactured by X-Rite, Inc.). The obtained OD value was divided by the film thickness of the cured film to provide an OD value per 1 µm (hereinafter, referred to as OD/µm) (OD value per 1 µm=OD value/film thickness of cured film).

### Synthesis Example 1: Synthesis of quinone diazide compound (c-1)

Under a dried nitrogen stream, TrisP-PA (trade name, manufactured by Honshu Kagaku Industry Co., Ltd.) (21.22 g (0.05 mol)) and 5-naphthoquinonediazide sulfonic acid chloride (26.87 g (0.10 mol)) were dissolved in 1,4-dioxane (450 g) at room temperature. To this solution was added dropwise a mixture of 1,4-dioxane (50 g) and triethylamine (15.18 g) while avoiding the increase in temperature of the inside of the system to 35°C or higher. After the dropwise addition, the resultant mixture was stirred at 30°C for 2 hours. The triethylamine salt was removed by filtration, the obtained filtrate was charged into water, and the precipitate was collected by filtration. The obtained precipitate was dried with a vacuum dryer to obtain a quinone diazide compound (c-1) represented by the following formula.

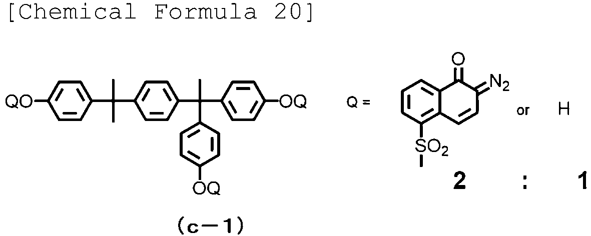

### Synthesis Example 2 Synthesis of polyhydroxystyrene (p-1)

To a mixed solution of 2400 g of tetrahydrofuran and 2.56 g (0.04 mol) of sec-butyllithium as an initiator, 105.75 g (0.6 mol) of p-t-butoxystyrene was added. The resultant mixture was then polymerized with stirring for 3 hours, and a polymerization termination reaction was performed by adding 12.82 g (0.4 mol) of methanol. Next, in order to purify the resultant polymer, the reaction mixture was poured into 3 L of methanol, and the precipitated polymer was then dried. The obtained polymer was dissolved in 1.6 L of acetone, 2 g of concentrated hydrochloric acid was added thereto at 60°C, followed by stirring for 7 hours to deprotect the p-t-butoxy group, and the p-t-butoxystyrene was converted into hydroxystyrene. After the completion of the reaction, the solution was poured into water to precipitate the polymer, and the obtained precipitate was cleaned with water 3 times and then dried in a vacuum dryer at 50°C for 24 hours to obtain the target polyhydroxystyrene (p-1).

### Synthesis Example 3 Synthesis of diamine compound (α)

18.3 g (0.05 mol) of 6FAP was dissolved in 100 mL of acetone and 17.4 g (0.3 mol) of propylene oxide and cooled to -15°C. A solution prepared by dissolving 3-nitrobenzoyl chloride (20.4 g (0.11 mol)) in acetone (100 mL) was added dropwise to the cooled solution. After the completion of the dropwise addition, the mixture was allowed to react at -15°C for 4 hours, and then the temperature was returned to room temperature. The precipitated white solid was separated by filtration and vacuum-dried at 50°C.

30 g of the obtained solid was placed in a 300-mL stainless steel autoclave, and then dispersed in 250 mL of methyl cellosolve, and 2 g of 5% by mass palladium on carbon was added thereto. Hydrogen was introduced thereinto with a balloon, and a reduction reaction was performed at room temperature. After about 2 hours, the reaction was completed when it was confirmed that the balloon could not deflate any more. After the completion of the reaction, the solution was filtered to remove the palladium compound used as a catalyst and concentrated with a rotary evaporator to obtain a fluorine atom-containing diamine compound (α) as represented by the following formula.

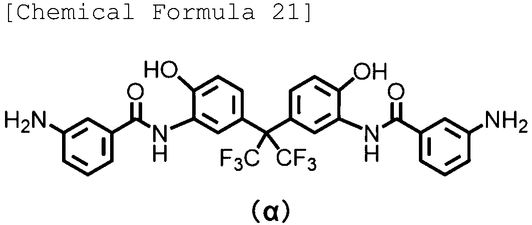

### Synthesis Example 4 Synthesis of diamine compound (β)

Into a 500-ml four-necked flask equipped with a stirrer, a thermocouple and a dropping funnel, 25.66 g (0.086 mol) of 4,4'-(2-ethylhexylidene)diphenol and 100 ml of glacial acetic acid were charged and stirred, and the internal temperature was raised to 50°C in a hot water bath. 2 ml (0.026 mol) of concentrated nitric acid was added dropwise thereto over 1 hour, and thereafter, the internal temperature was lowered to 13°C by ice cooling, and 13.3 ml (0.149 mol) of concentrated nitric acid was further added dropwise over 1 hour. Thereafter, stirring was continuously conducted for 3 hours, and the precipitated yellow crystals were collected by filtration, cleaned sequentially with 40 ml of glacial acetic acid and 80 ml of deionized water, and dried under reduced pressure to obtain a dinitro form.

Subsequently, 52.44 g (0.135 mol) of the dinitro form, 180 ml (3.71 mol) of hydrazine monohydrate and 900 ml of ethanol were put in a 2-L four-necked flask equipped with a stirrer, a thermocouple, a Dimroth cooling tube and a dropping funnel, and stirred under ice cooling, and 0.9 g of 5% palladium on carbon (manufactured by Wako Pure Chemical Industries, Ltd.) suspended in 30 ml of ethanol was added dropwise thereto over 1 hour. Thereafter, the solution was refluxed for 2 hours, and the palladium on carbon was removed by filtration while cleaning with 300 ml of ethanol. All of the solvent was removed by heating under reduced pressure conditions. The residue was cleaned with 75 ml of ethanol cooled with ice and filtered, then further cleaned sequentially with 75 ml of deionized water and 150 ml of diethyl ether, and dried under reduced pressure to obtain a diamine (β).

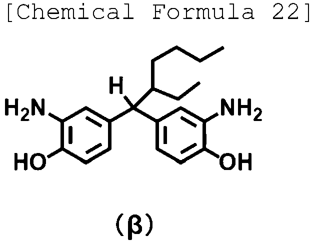

### Synthesis Example 5 Synthesis of diamine compound (γ)

A diamine compound (γ) was obtained in the same manner as in Synthesis Example 3 except that 16.4 g (0.05 mol) of the diamine compound (β) obtained in Synthesis Example 4 was used in place of 6FAP.

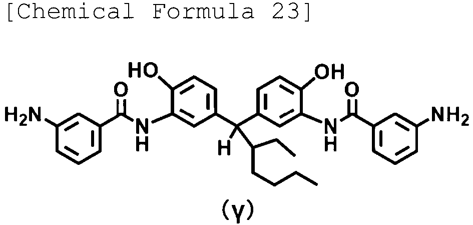

### Synthesis Example 6 Synthesis of diamine compound

### (δ)

A diamine compound (δ) was obtained in the same manner as in Synthesis Example 4 except that 26.70 g (0.086 mol) of 4,4'-(3,3,5-trimethylcyclohexylidene)bisphenol) was used in place of 4,4'-(2-ethylhexylidene)diphenol) to synthesize a dinitro form, and 54.06 g (0.135 mol) of the dinitro form was used.

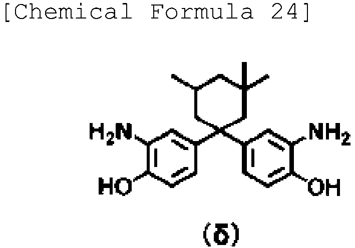

### Synthesis Example 7 Synthesis of alkali-soluble resin (a-1)

Under a dry nitrogen stream, 31.02 g (0.10 mol) of ODPA was dissolved in 500 g of MPA. To this solution, 21.96 g (0.085 mol) of BAP and 1.24 g (0.005 mol) of SiDA were added together with 50 g of MPA, and the solution was allowed to react for 2 hours at 40°C. Next, 2.18 g (0.02 mol) of 3-aminophenol that served as an end-sealing agent was added together with 5 g of MPA to the reaction solution, and the resultant mixture was allowed to react at 50°C for 2 hours. Then, a solution prepared by diluting 32.39 g (0.22 mol) of N,N-dimethylformamide diethyl acetal with 50 g of MPA was charged. After the charge, the solution was stirred at 50°C for 3 hours. After the completion of the stirring, the solution was cooled to room temperature and the solution was charged into 3 L of water to obtain a white precipitate. The obtained precipitate was collected by filtration, cleaned with water three times, and then dried in a vacuum dryer at 80°C for 24 hours, thereby obtaining a polyimide precursor (a-1) which was an alkali-soluble resin.

### Synthesis Example 17 Synthesis of alkali-soluble resin (a-11)

Under a dry nitrogen stream, 12.92 g (0.050 mol) of BAP, 12.82 g (0.035 mol) of 6FAP, 1.24 g (0.005 mol) of SiDA, 2.18 g (0.02 mol) of MAP, and 52.8 g (0.6 mol) of glycidyl methyl ether were dissolved in 500 g of MPA, and the temperature of the solution was cooled to -15°C. A solution prepared by dissolving 29.51 g (0.10 mol) of OBBC in 50 g of MPA was added dropwise thereto so that an internal temperature did not exceed 0°C. After the completion of the dropwise addition, stirring was continuously conducted at -15°C for 6 hours. After the completion of the reaction, the solution was charged into 3 L of water containing 10% by weight of methanol and a white precipitate was collected. The obtained precipitate was collected by filtration, cleaned with water three times, and then dried in a vacuum dryer at 80°C for 24 hours, thereby obtaining a polybenzoxazole precursor (a-11) which was an alkali-soluble resin.

### Synthesis Examples 8 to 16 and 18 to 25, and Comparative Synthesis Examples 1 to 4

Polyimide precursors (a-2) to (a-10), (a-12) to (a-19), and (a'-1) to (a'-4) were obtained in the same manner as in Synthesis Example 7 except that the amine component, the acid component, and the polymerization solvent were changed as shown in Table 1.

### Comparative Synthesis Example 5

A polybenzoxazole precursor (a'-5) was obtained in the same manner as in Synthesis Example 17 except that the amine component, the acid component, and the polymerization solvent were changed as shown in Table 1.

The names of the compounds used for each of Examples and Comparative Examples are shown below. Compounds other than commercially available products were synthesized using known methods.
GBL: γ-butyrolactone
MPA: 3-methoxy-N,N-dimethylpropanamide
NMP: N-methylpyrrolidone
BAP: 2,2-bis(3-amino-4-hydroxyphenyl)propane (diamine containing no fluorine atom)
ABPS: bis(3-amino-4-hydroxyphenyl)sulfone (diamine containing no fluorine atom)
BAHF: 9,9-bis(3-amino-4-hydroxyphenyl)fluorene (diamine containing no fluorine atom and having fluorene structure)
SiDA: 1,3-bis(3-aminopropyl)tetramethyldisiloxane (diamine containing no fluorine atom and containing silicon atom)
6FAP: 2,2-bis(3-amino-4-hydroxyphenyl)hexafluoropropane (diamine containing fluorine atom)
8FBZ: 4,4'-diaminooctafluorobiphenyl (diamine containing fluorine atom directly bonded to sp2 carbon)
MAP: 3-aminophenol
ODPA: 3,3',4,4'-diphenyl ether tetracarboxylic dianhydride (acid dianhydride containing no fluorine atom)
TDA: tetralin dianhydride (acid dianhydride containing no fluorine atom and containing alicyclic structure)
6FDA: 2,2-bis(3,4-dicarboxyphenyl)hexafluoropropane dianhydride (acid dianhydride containing fluorine atom)
OBBC: diphenyl ether dicarboxylic acid dichloride (acid chloride containing no fluorine atom)
b-1: 1,2,4-trihydroxybenzene (aromatic hydrocarbon compound having at least three phenolic hydroxyl groups in one aromatic ring)
b'-1: 1,1,1-tris(4-hydroxyphenyl)ethane
BTS: butyl p-toluenesulfonate (thermal acid generator that generates specific sulfonic acid and/or sulfonate ions during heat-treatment)
s-1: 3-glycidoxypropyltrimethoxysilane (silane coupling agent)
e-1: thermal crosslinking agent represented by structure shown below
e-2: thermal crosslinking agent represented by structure shown below
d1-1: dye represented by structure shown below
d1-2: dye represented by structure shown below

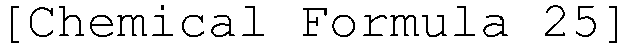

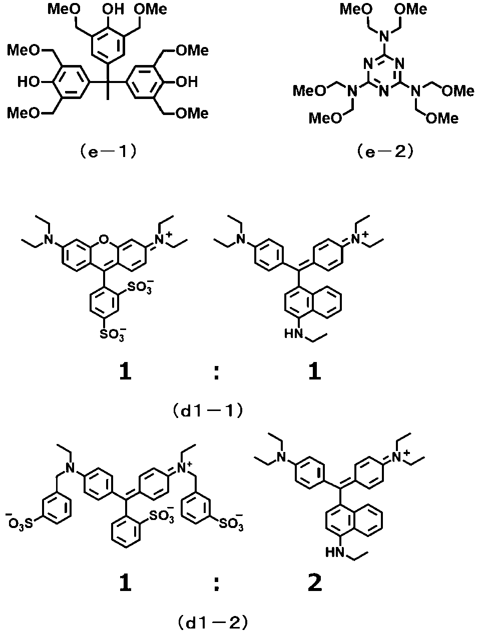

### Example 1

In 50 g of GBL, 10.0 g of a polyimide precursor (a-1), 2.0 g of a photosensitive compound (c-1), and 2.0 g of (e-1) were dissolved, and then the resultant solution was filtered through a 0.2 µm polytetrafluoroethylene filter to obtain a varnish A-1 of a positive-type photosensitive resin composition. Using the obtained varnish A-1, the electron probe microanalyzer measurement of the cured film, the evaluation of the reworkability during the preparation of the cured film, the evaluation of the bending resistance of the cured film, the calculation of the content of β-alkoxypropionamide in the cured film, the evaluation of the long-term reliability of the organic EL display device, the evaluation of the light shielding property at 450 nm, and the evaluation of the visible light shielding property were performed as described above.

The cured film contains a compound derived from a quinone diazide compound (c-1), that is, one or more selected from the group consisting of a carboxylic acid having an indene structure, a carboxylic acid ester having an indene structure, a sulfonic acid having an indene structure, and a sulfonic acid aryl ester having an indene structure. This can be confirmed by analyzing the cured film by methods such as proton nuclear magnetic resonance spectroscopy, infrared spectroscopy, pyrolysis gas chromatography-mass spectrometry, reactive pyrolysis gas chromatography-mass spectrometry, liquid chromatography-mass spectrometry, and time-of-flight secondary ion mass spectrometry.

### Examples 2 to 33, Comparative Examples 1 to 5

Varnishes of photosensitive resin compositions were obtained in the same manner as in Example 1 except that a component (a), a component (b), a component (c), other components, and a solvent were changed as shown in Tables 2 and 3. Using the obtained varnishes, the electron probe microanalyzer measurement of the cured film, the evaluation of the reworkability during the preparation of the cured film, the evaluation of the bending resistance of the cured film, the calculation of the content of β-alkoxypropionamide in the cured film, the evaluation of the long-term reliability of the organic EL display device, the evaluation of the light shielding property at 450 nm, and the evaluation of the visible light shielding property were performed as described above. A compound derived from a quinone diazide compound (c-1) in the cured film was the same as that in Example 1.

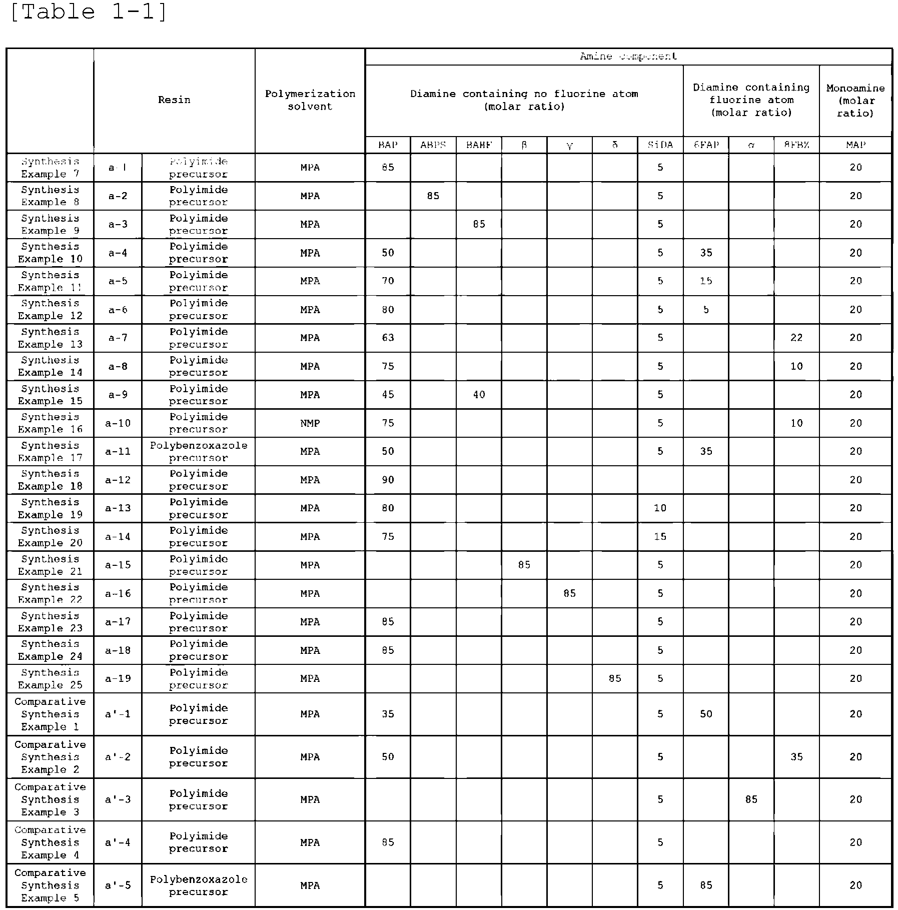

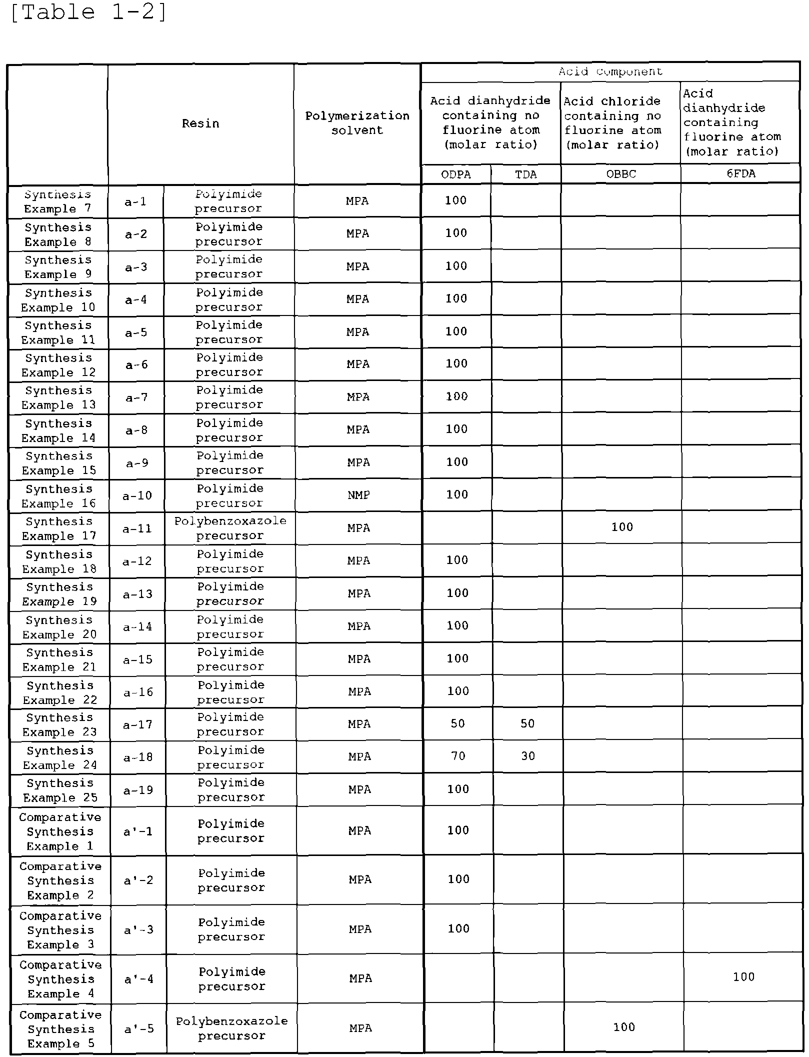

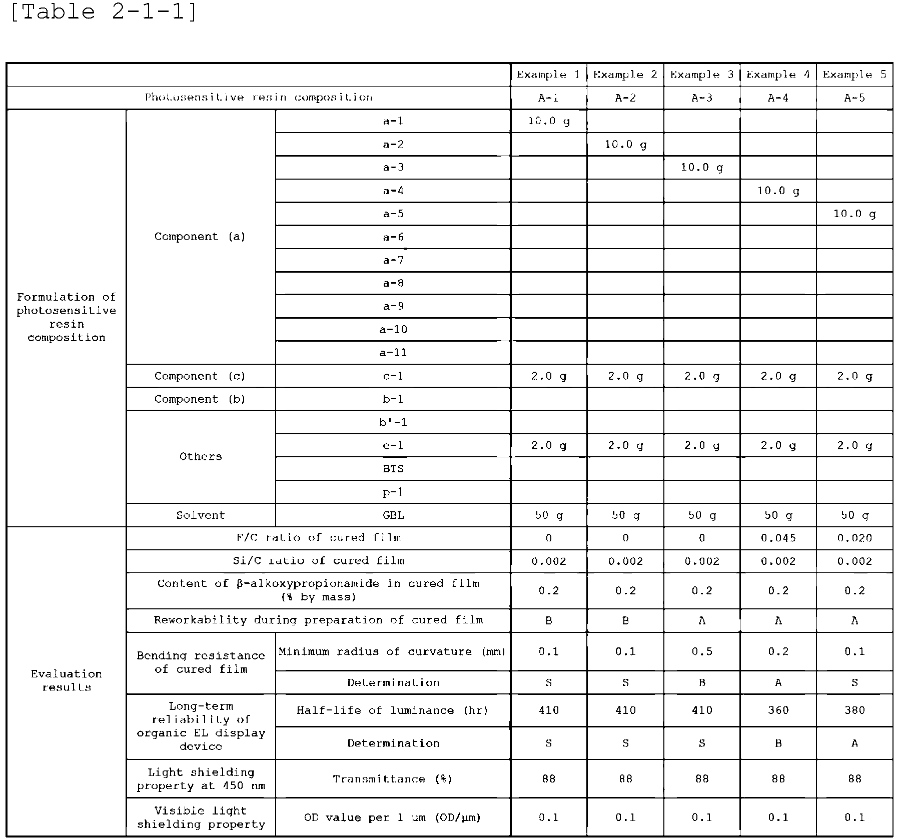

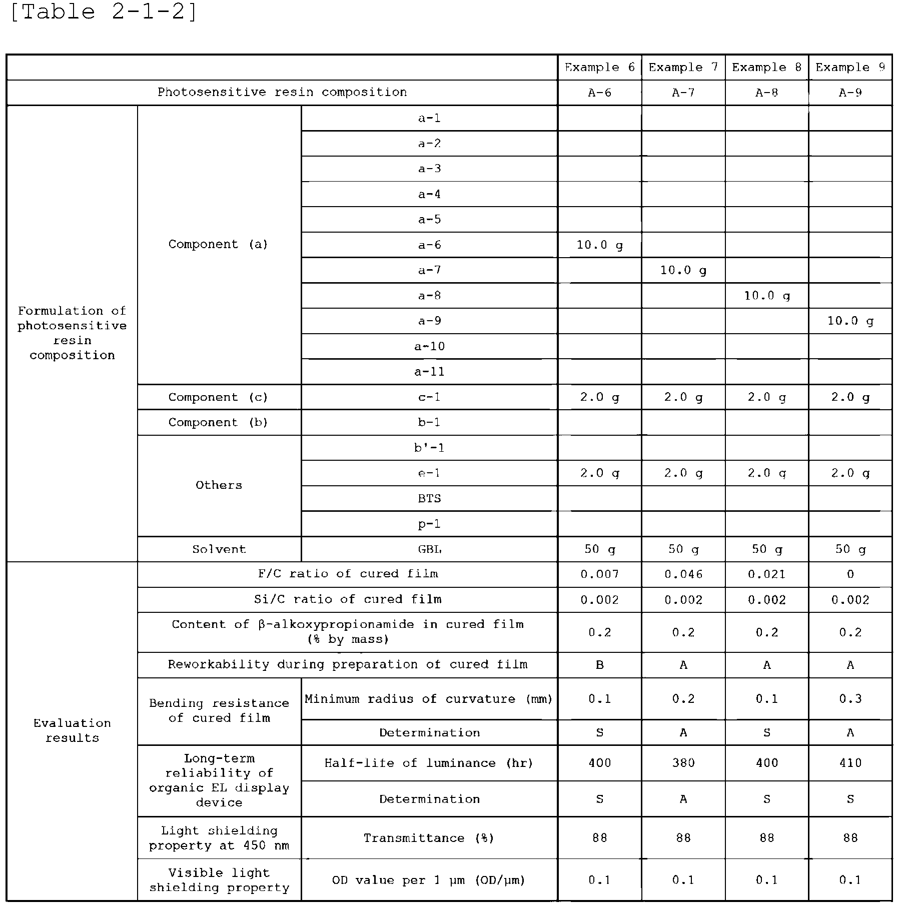

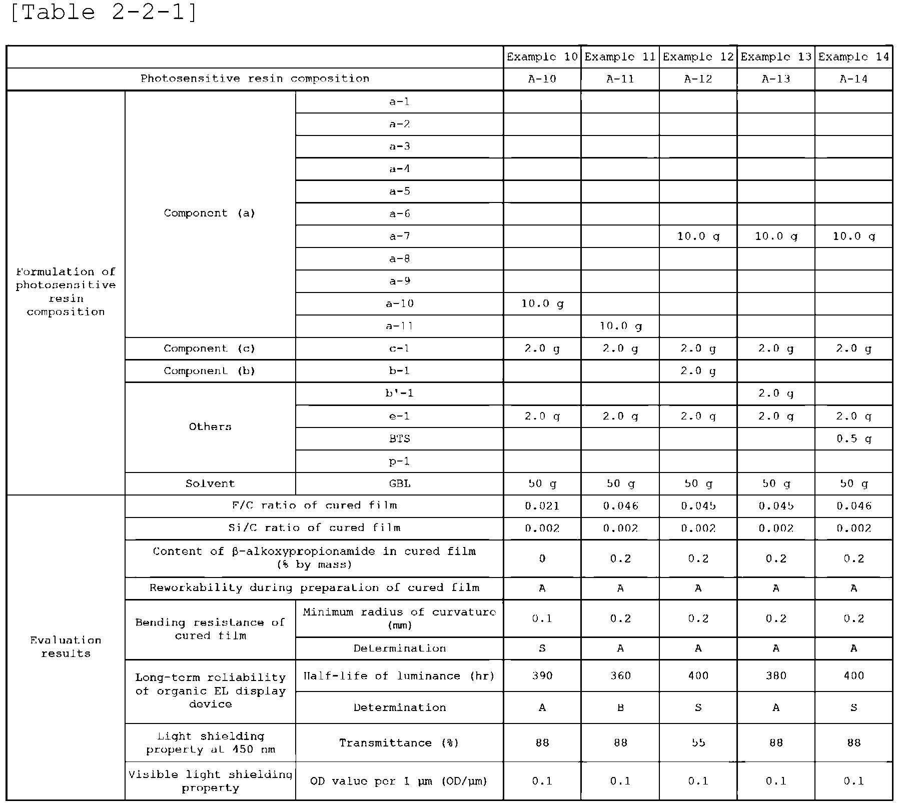

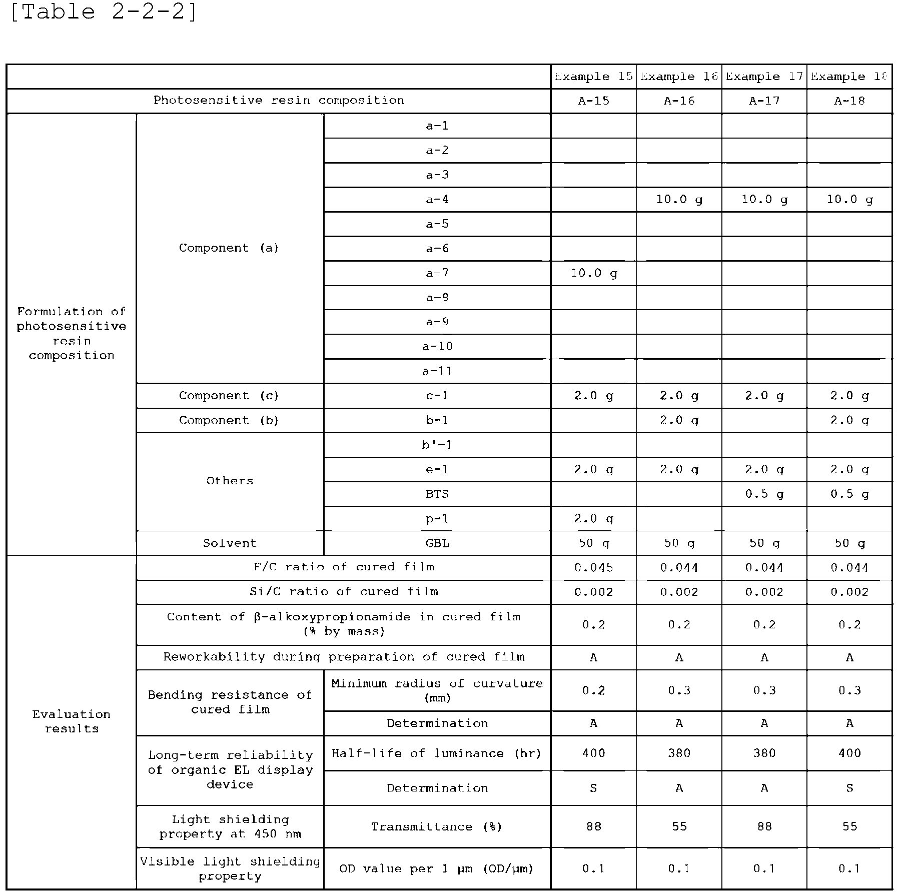

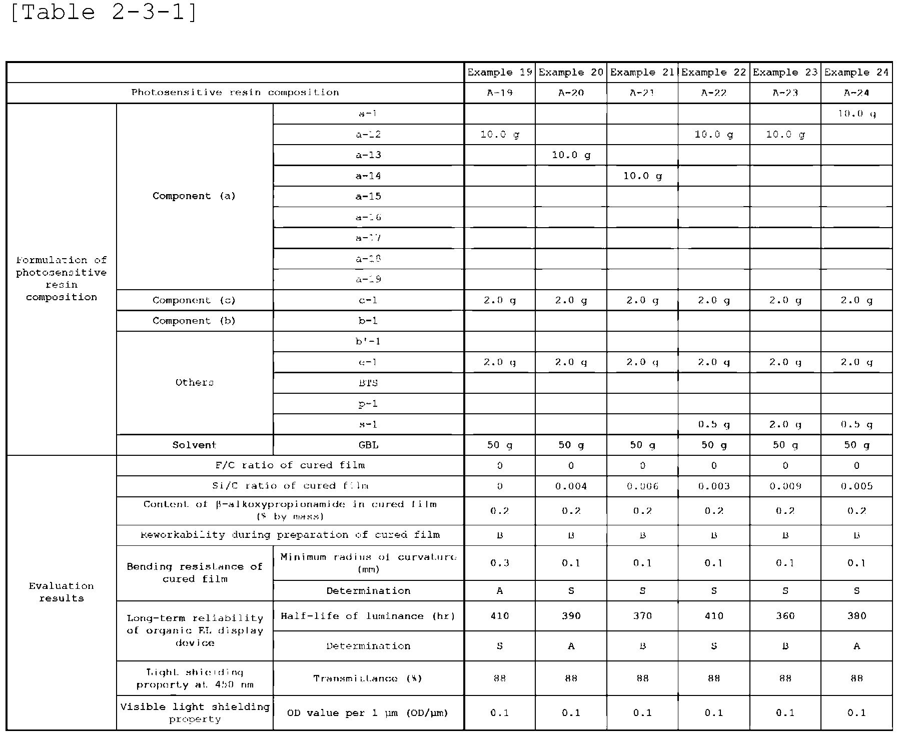

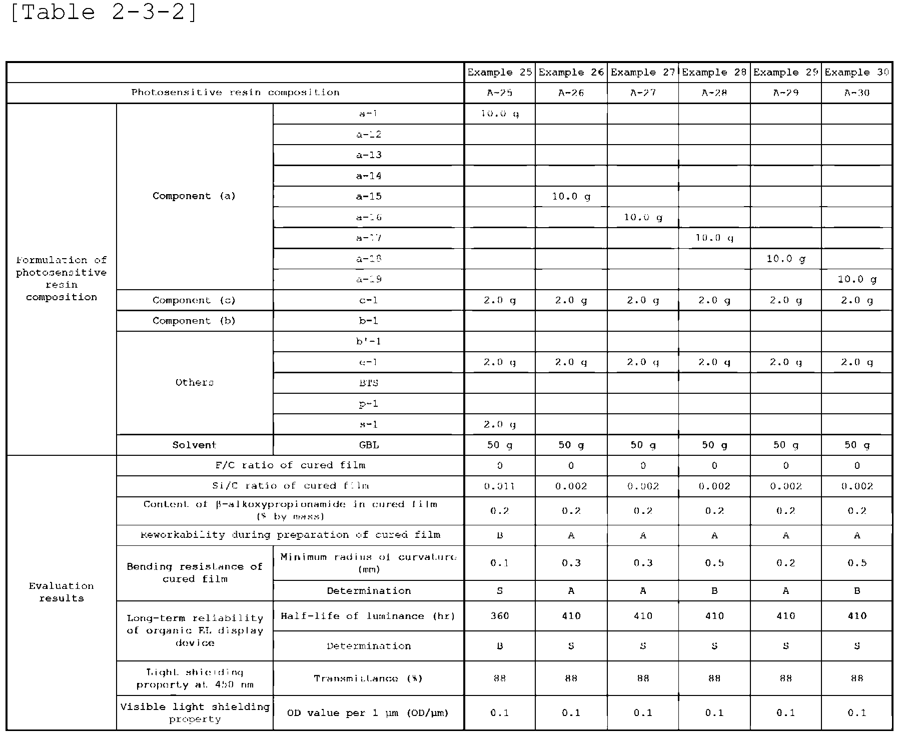

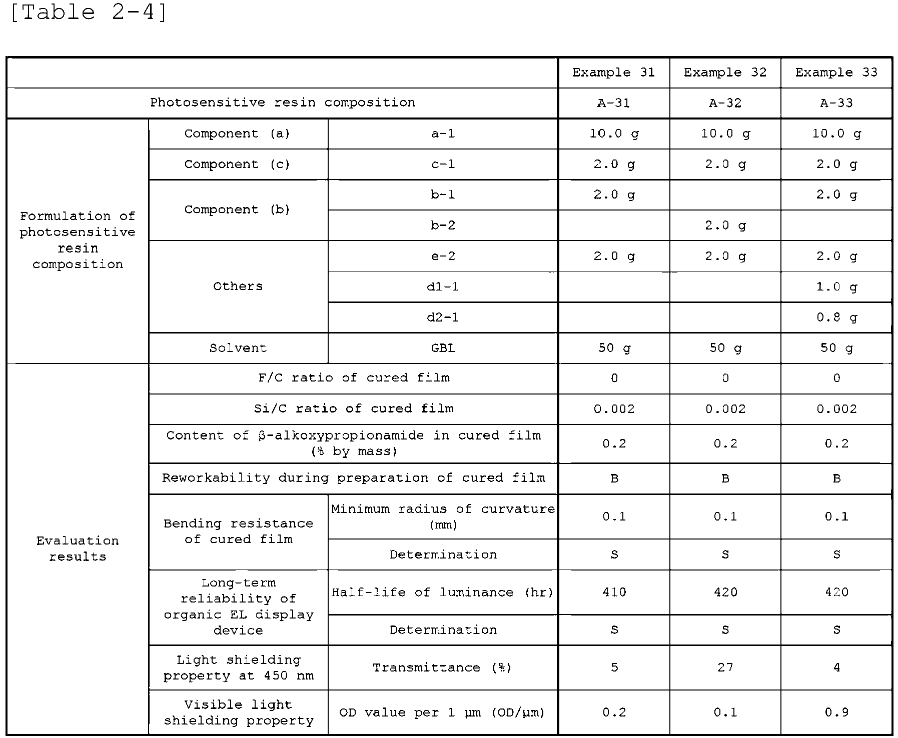

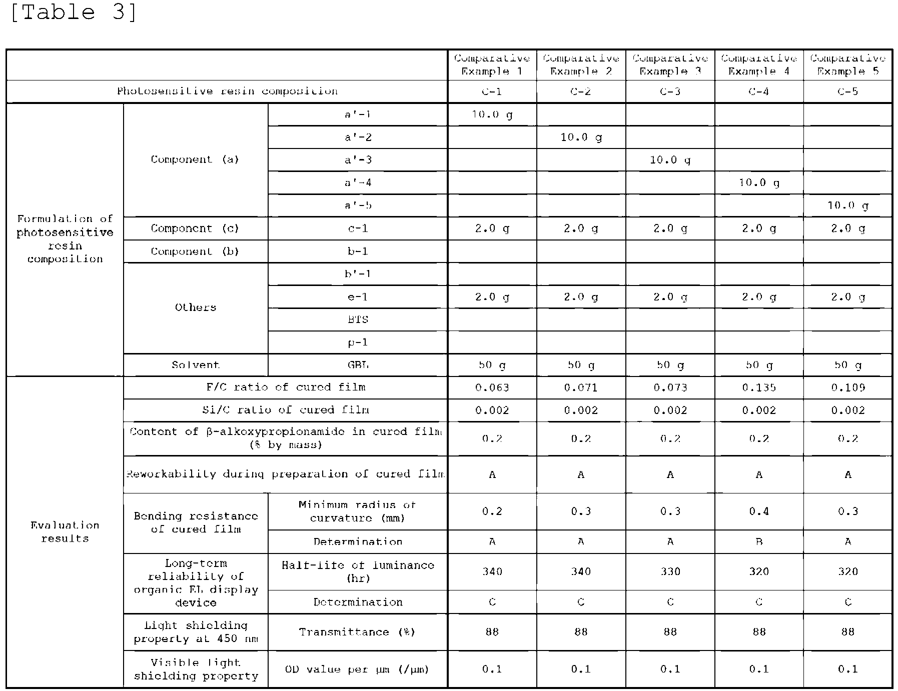

### DESCRIPTION OF REFERENCE SIGNS

- 1:: Substrate
- 2:: TFT
- 3:: TFT insulation layer
- 4:: Wiring
- 5:: Planarization layer
- 6:: Contact hole
- 7:: First electrode
- 8:: Pixel division layer
- 9:: Organic EL layer
- 10:: Second electrode
- 11:: Display device
- 12:: Light-emitting element
- 13:: Cured film
- 14,: 14c: Metal wiring
- 15:: Counter substrate
- 16:: Electrode terminal
- 17:: Light-emitting element drive substrate
- 18:: Drive element
- 19:: Barrier metal
- 20:: Solder bump
- 21:: Non-alkali glass substrate
- 22:: First electrode (transparent electrode)
- 23:: Auxiliary electrode
- 24:: Pixel division layer
- 25:: Organic EL layer
- 26:: Second electrode (non-transparent electrode)

## Claims

1. A cured film comprising a polyimide and/or a polybenzoxazole, wherein
a molar ratio F (fluorine atom)/C (carbon atom) of a fluorine atom to a carbon atom obtained when a cross section of the cured film is measured with an electron probe microanalyzer is 0 or more and 0.05 or less.

2. The cured film according to claim 1, further comprising one or more compounds selected from the group consisting of a carboxylic acid having an indene structure, a carboxylic acid ester having an indene structure, a sulfonic acid having an indene structure, and a sulfonic acid aryl ester having an indene structure.

3. The cured film according to claim 1 or 2, further comprising polyhydroxystyrene and/or a polyhydroxystyrene/polystyrene copolymer.

4. The cured film according to claim 1 or 2, further comprising an aromatic hydrocarbon compound having at least three phenolic hydroxyl groups in one aromatic ring.

5. The cured film according to claim 1 or 2, further comprising β-alkoxypropionamide.

6. The cured film according to claim 1 or 2, further comprising one or more sulfonic acids selected from the group consisting of compounds represented by any of the formulas (3) to (5) or sulfonate ions derived from one or more sulfonic acids selected from the group consisting of compounds represented by any of the formulas (3) to (5): 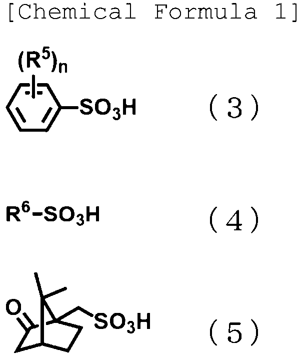 wherein
in the formula (3), R⁵ each independently represents a hydrogen atom or a monovalent organic group having 1 to 10 carbon atoms, and n represents an integer of 1 to 5; and
in the formula (4), R⁶ represents a monovalent organic group having 1 to 10 carbon atoms.

7. The cured film according to claim 1 or 2, wherein
the polyimide and/or the polybenzoxazole has repeating units each composed of an acid component and an amine component, and
when the acid component and the amine component in all the repeating units are each 100 mol%, the total of the acid component having a fluorene structure and the amine component having a fluorene structure is 0 mol% or more and 50 mol% or less with respect to 200 mol% of the total of the acid component and the amine component.

8. The cured film according to claim 1 or 2, wherein the polyimide and/or the polybenzoxazole has a residue represented by the formula (6): 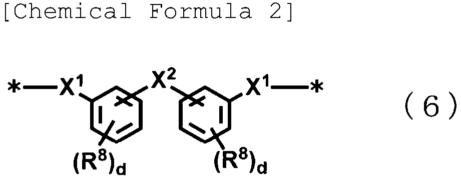 wherein
in the formula (6), X¹ each independently represents a direct bond, a divalent group represented by the formula (7) or a divalent group represented by the formula (8), X² represents a divalent group represented by the formula (9) or the formula (10), R⁸ each independently represents an alkyl group having 1 to 4 carbon atoms or a hydroxyl group, and d each independently represents an integer of 0 to 4; 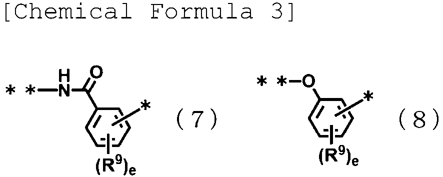
in the formulas (7) and (8), R⁹ each independently represents an alkyl group having 1 to 4 carbon atoms or a hydroxyl group, e each independently represents an integer of 0 to 4, * represents * in the formula (6), ** represents a bonding point bonded to an aromatic ring; 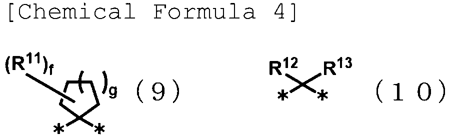
in the formulas (9) and (10), R¹¹ each independently represents an alkyl group having 1 to 4 carbon atoms, R¹² and R¹³ each independently represent a hydrocarbon group having 1 to 10 carbon atoms or a hydrogen atom, * represents a bonding point bonded to an aromatic ring, f each independently represents an integer of 1 to 4, g represents 1 or 2, provided that R¹² and R¹³ represent different substituents.

9. The cured film according to claim 1 or 2, wherein
the polyimide and/or the polybenzoxazole has repeating units each composed of an acid component and an amine component, and
when the acid component and the amine component in all the repeating units are each 100 mol%, the total of the acid component having an alicyclic structure and the amine component having an alicyclic structure is 0 mol% or more and 40 mol% or less with respect to 200 mol% of the total of the acid component and the amine component.

10. The cured film according to claim 1 or 2, wherein the polyimide and/or the polybenzoxazole contains a repeating unit having a fluorine atom directly bonded to an sp2 carbon.

11. The cured film according to claim 10, wherein
the polyimide and/or the polybenzoxazole has repeating units each composed of an acid component and an amine component, and
when the acid component and the amine component in all the repeating units are each 100 mol%, the total of the acid component having a fluorine atom directly bonded to the sp2 carbon and the amine component having a fluorine atom directly bonded to the sp2 carbon is 5 mol% or more and 100 mol% or less with respect to 200 mol% of the total of the acid component and the amine component.

12. The cured film according to claim 1 or 2, wherein a molar ratio Si (silicon atom)/C (carbon atom) of a silicon atom to a carbon atom obtained when a cross section of the cured film is measured with an electron probe microanalyzer is 0.001 or more and 0.005 or less.

13. The cured film according to claim 1 or 2, wherein the cured film has a transmittance of light at a wavelength of 450 nm per film thickness of 2.0 µm of 30% or less.

14. The cured film according to claim 1 or 2, wherein the cured film has an OD value of 0.5 to 1.5 in visible light per film thickness of 1 um.

15. An organic EL display device comprising the cured film according to claim 1 or 2.

16. The organic EL display device according to claim 15, further comprising a color filter having a black matrix.

17. An electronic component comprising the cured film according to claim 1 or 2.
